# EUROPEAN PATENT APPLICATION

(11) **EP 4 056 696 A1**
(43) Date of publication of application: **14.09.2022**
(21) Application number: 20885771.4
(22) Date of filing: 06.11.2020
(51) Int. Cl.: C12N 9/50, A61P 25/28, A61K 38/00

(54) **SYNTHETIC ALPHA-SECRETASE AND USE THEREOF**

(30) Priority: 08.11.2019 KR 20190142615
(71) Applicant: Gwangju Institute of Science and Technology, Gwangju 61005 (KR); Industry Foundation of Chonnam National University, Gwangju 61186 (KR)
(72) Inventor: PARK, Woo Jin, Gwangju 61005 (KR); KIM, Sung Bin, Gwangju 61005 (KR); KIM, Sung Yun, Gwangju 61005 (KR); CHOI, Won Seok, Gwangju 61186 (KR)
(74) Representative: Dehns
(86) International application number: PCT/KR2020/015531
(87) International publication number: WO 2021/091317

(57) **Abstract**

The present invention relates to synthetic α-secretase (SAS) and a use thereof. According to the present invention, a synthetic α-secretase, which is a fusion protein comprising, as an active ingredient, NIa protease, a fragment thereof or a variant thereof, can inhibit the formation of amyloid β, degrade extracellularly secreted amyloid β, and degrade amyloid β internalized in cells. Therefore, the present invention allows intracellular/extracellular degradation of amyloid β, which is the cause of various diseases including Alzheimer's disease, and thus can be usable in the prevention or treatment of such diseases.

<110>    GWANGJU INSTITUTE OF SCIENCE AND TECHNOLOGY
         INDUSTRY FOUNDATION OF CHONNAM NATIONAL UNIVERSITY

<120>    SYNTHETIC ALPHA-SECRETASE AND USE THEREOF

<130>    PCC010098GIS

<150>    KR 10-2019-0142615
<151>    2019-11-08

<160>    51

<170>    KoPatentIn 3.0

<210>    1
<211>    501
<212>    PRT
<213>    Artificial Sequence

<220>
<223>    Human beta-secretase


<400>    1
Met Ala Gln Ala Leu Pro Trp Leu Leu Leu Trp Met Gly Ala Gly Val
  1               5                  10                  15

Leu Pro Ala His Gly Thr Gln His Gly Ile Arg Leu Pro Leu Arg Ser
             20                  25                  30

Gly Leu Gly Gly Ala Pro Leu Gly Leu Arg Leu Pro Arg Glu Thr Asp
         35                  40                  45

Glu Glu Pro Glu Glu Pro Gly Arg Arg Gly Ser Phe Val Glu Met Val
     50                  55                  60

Asp Asn Leu Arg Gly Lys Ser Gly Gln Gly Tyr Tyr Val Glu Met Thr
 65                  70                  75                  80

Val Gly Ser Pro Pro Gln Thr Leu Asn Ile Leu Val Asp Thr Gly Ser
                 85                  90                  95

Ser Asn Phe Ala Val Gly Ala Ala Pro His Pro Phe Leu His Arg Tyr
            100                 105                 110

Tyr Gln Arg Gln Leu Ser Ser Thr Tyr Arg Asp Leu Arg Lys Gly Val
        115                 120                 125

Tyr Val Pro Tyr Thr Gln Gly Lys Trp Glu Gly Glu Leu Gly Thr Asp
    130                 135                 140

Leu Val Ser Ile Pro His Gly Pro Asn Val Thr Val Arg Ala Asn Ile
145                 150                 155                 160

Ala Ala Ile Thr Glu Ser Asp Lys Phe Phe Ile Asn Gly Ser Asn Trp
                165                 170                 175

Glu Gly Ile Leu Gly Leu Ala Tyr Ala Glu Ile Ala Arg Pro Asp Asp
            180                 185                 190

Ser Leu Glu Pro Phe Phe Asp Ser Leu Val Lys Gln Thr His Val Pro
        195                 200                 205

Asn Leu Phe Ser Leu Gln Leu Cys Gly Ala Gly Phe Pro Leu Asn Gln
    210                 215                 220

Ser Glu Val Leu Ala Ser Val Gly Gly Ser Met Ile Ile Gly Gly Ile
225                 230                 235                 240

Asp His Ser Leu Tyr Thr Gly Ser Leu Trp Tyr Thr Pro Ile Arg Arg
                245                 250                 255

Glu Trp Tyr Tyr Glu Val Ile Ile Val Arg Val Glu Ile Asn Gly Gln
            260                 265                 270

Asp Leu Lys Met Asp Cys Lys Glu Tyr Asn Tyr Asp Lys Ser Ile Val
        275                 280                 285

Asp Ser Gly Thr Thr Asn Leu Arg Leu Pro Lys Lys Val Phe Glu Ala
    290                 295                 300

Ala Val Lys Ser Ile Lys Ala Ala Ser Ser Thr Glu Lys Phe Pro Asp
305                 310                 315                 320

Gly Phe Trp Leu Gly Glu Gln Leu Val Cys Trp Gln Ala Gly Thr Thr
                325                 330                 335

Pro Trp Asn Ile Phe Pro Val Ile Ser Leu Tyr Leu Met Gly Glu Val
            340                 345                 350

Thr Asn Gln Ser Phe Arg Ile Thr Ile Leu Pro Gln Gln Tyr Leu Arg
        355                 360                 365

Pro Val Glu Asp Val Ala Thr Ser Gln Asp Asp Cys Tyr Lys Phe Ala
    370                 375                 380

Ile Ser Gln Ser Ser Thr Gly Thr Val Met Gly Ala Val Ile Met Glu
385                 390                 395                 400

Gly Phe Tyr Val Val Phe Asp Arg Ala Arg Lys Arg Ile Gly Phe Ala
                405                 410                 415

Val Ser Ala Cys His Val His Asp Glu Phe Arg Thr Ala Ala Val Glu
            420                 425                 430

Gly Pro Phe Val Thr Leu Asp Met Glu Asp Cys Gly Tyr Asn Ile Pro
        435                 440                 445

Gln Thr Asp Glu Ser Thr Leu Met Thr Ile Ala Tyr Val Met Ala Ala
    450                 455                 460

Ile Cys Ala Leu Phe Met Leu Pro Leu Cys Leu Met Val Cys Gln Trp
465                 470                 475                 480

Arg Cys Leu Arg Cys Leu Arg Gln Gln His Asp Asp Phe Ala Asp Asp
                485                 490                 495

Ile Ser Leu Leu Lys
            500


<210>    2
<211>    1506
<212>    DNA
<213>    Artificial Sequence

<220>
<223>    Human beta-secretase (cDNA)


<400>    2
atggcccaag ccctgccctg gctcctgctg tggatgggcg cgggagtgct gcctgcccac      60

ggcacccagc acggcatccg gctgcccctg cgcagcggcc tggggggcgc cccccctggg     120

ctgcggctgc cccgggagac cgacgaagag cccgaggagc ccggccggag gggcagcttt     180

gtggagatgg tggacaacct gaggggcaag tcggggcagg gctactacgt ggagatgacc     240

gtgggcagcc ccccgcagac gctcaacatc ctggtggata caggcagcag taactttgca     300

gtgggtgctg ccccccaccc cttcctgcat cgctactacc agaggcagct gtccagcaca     360

taccgggacc tccggaaggg tgtgtatgtg ccctacaccc agggcaagtg ggaaggggag     420

ctgggcaccg acctggtaag catcccccat ggccccaacg tcactgtgcg tgccaacatt     480

gctgccatca ctgaatcaga caagttcttc atcaacggct ccaactggga aggcatcctg     540

gggctggcct atgctgagat tgccaggcct gacgactccc tggagccttt ctttgactct     600

ctggtaaagc agacccacgt tcccaacctc ttctccctgc agctttgtgg tgctggcttc     660

cccctcaacc agtctgaagt gctggcctct gtcggaggga gcatgatcat tggaggtatc     720

gaccactcgc tgtacacagg cagtctctgg tatacaccca tccggcggga gtggtattat     780

gaggtcatca ttgtgcgggt ggagatcaat ggacaggatc tgaaaatgga ctgcaaggag     840

tacaactatg acaagagcat tgtggacagt ggcaccacca accttcgttt gcccaagaaa     900

gtgtttgaag ctgcagtcaa atccatcaag gcagcctcct ccacggagaa gttccctgat     960

ggtttctggc taggagagca gctggtgtgc tggcaagcag gcaccacccc ttggaacatt    1020

ttcccagtca tctcactcta cctaatgggt gaggttacca accagtcctt ccgcatcacc    1080

atccttccgc agcaatacct gcggccagtg gaagatgtgg ccacgtccca agacgactgt    1140

tacaagtttg ccatctcaca gtcatccacg ggcactgtta tgggagctgt tatcatggag    1200

ggcttctacg ttgtctttga tcgggcccga aaacgaattg gctttgctgt cagcgcttgc    1260

catgtgcacg atgagttcag gacggcagcg gtggaaggcc cttttgtcac cttggacatg    1320

gaagactgtg gctacaacat tccacagaca gatgagtcaa ccctcatgac catagcctat    1380

gtcatggctg ccatctgcgc cctcttcatg ctgccactct gcctcatggt gtgtcagtgg    1440

cgctgcctcc gctgcctgcg ccagcagcat gatgactttg ctgatgacat ctccctgctg    1500

aagtga                                                               1506


<210>    3
<211>    21
<212>    PRT
<213>    Artificial Sequence

<220>
<223>    Human beta-secretase signal sequence

<400>    3
Met Ala Gln Ala Leu Pro Trp Leu Leu Leu Trp Met Gly Ala Gly Val
1               5                   10                  15

Leu Pro Ala His Gly
            20

<210>    4
<211>    34
<212>    PRT
<213>    Artificial Sequence

<220>
<223>    Human beta-secretase pro-domain

<400>    4
Thr Gln His Gly Ile Arg Leu Pro Leu Arg Ser Gly Leu Gly Gly Ala
1               5                   10                  15

Pro Leu Gly Leu Arg Leu Pro Arg Glu Thr Asp Glu Glu Pro Glu Glu
            20                  25                  30

Pro Gly

<210>    5
<211>    22
<212>    PRT
<213>    Artificial Sequence

<220>
<223>    L1

<400>    5
Gly Thr Asp Leu Val Ser Ile Pro His Gly Pro Asn Val Thr Val Arg
1               5                   10                  15

Ala Asn Ile Ala Ala Ile
            20

<210>    6
<211>    20
<212>    PRT
<213>    Artificial Sequence

<220>
<223>    L1

<400>    6
Asp Leu Val Ser Ile Pro His Gly Pro Asn Val Thr Val Arg Ala Asn
1               5                   10                  15

Ile Ala Ala Ile

            20

<210> 7
<211> 18
<212> PRT
<213> Artificial Sequence

<220>
<223> L1

<400> 7
Asp Leu Val Ser Ile Pro His Gly Pro Asn Val Thr Val Arg Ala Asn
1               5                   10                  15

Ile Ala

<210> 8
<211> 16
<212> PRT
<213> Artificial Sequence

<220>
<223> L1

<400> 8
Val Ser Ile Pro His Gly Pro Asn Val Thr Val Arg Ala Asn Ile Ala
1               5                   10                  15

<210> 9
<211> 14
<212> PRT
<213> Artificial Sequence

<220>
<223> L1

<400> 9
Ile Pro His Gly Pro Asn Val Thr Val Arg Ala Asn Ile Ala
1               5                   10

<210> 10
<211> 12
<212> PRT
<213> Artificial Sequence

<220>
<223> L1

<400> 10
Ile Pro His Gly Pro Asn Val Thr Val Arg Ala Asn
1               5                   10

<210>    11
<211>    10
<212>    PRT
<213>    Artificial Sequence

<220>
<223>    L1

<400>    11
His Gly Pro Asn Val Thr Val Arg Ala Asn
1               5                   10

<210>    12
<211>    243
<212>    PRT
<213>    Artificial Sequence

<220>
<223>    TuMV NIa protease protein

<400>    12
Ser Asn Ser Met Phe Arg Gly Leu Arg Asp Tyr Asn Pro Ile Ser Asn
1               5                   10                  15

Asn Ile Cys His Leu Thr Asn Val Ser Asp Gly Ala Ser Asn Ser Leu
            20                  25                  30

Tyr Gly Val Gly Phe Gly Pro Leu Ile Leu Thr Asn Arg His Leu Phe
        35                  40                  45

Glu Arg Asn Asn Gly Glu Leu Val Ile Lys Ser Arg His Gly Glu Phe
    50                  55                  60

Val Ile Lys Asn Thr Thr Gln Leu His Leu Leu Pro Ile Pro Asp Arg
65                  70                  75                  80

Asp Leu Leu Leu Ile Arg Leu Pro Lys Asp Ile Pro Pro Phe Pro Gln
                85                  90                  95

Lys Leu Gly Phe Arg Gln Pro Glu Lys Gly Glu Arg Ile Cys Met Val
            100                 105                 110

Gly Ser Asn Phe Gln Thr Lys Ser Ile Thr Ser Val Val Ser Glu Thr
        115                 120                 125

Ser Thr Ile Met Pro Val Glu Asn Ser Gln Phe Trp Lys His Trp Ile
    130                 135                 140

Ser Thr Lys Asp Gly Gln Cys Gly Ser Pro Met Val Ser Thr Lys Asp
145                 150                 155                 160

Gly Lys Ile Leu Gly Leu His Ser Leu Ala Asn Phe Gln Asn Ser Ile
                165                 170                 175

Asn Tyr Phe Ala Ala Phe Pro Asp Asp Phe Ala Glu Lys Tyr Leu His
            180                 185                 190

Thr Ile Glu Ala His Glu Trp Val Lys His Trp Lys Tyr Asn Thr Ser
        195                 200                 205

Ala Ile Ser Trp Gly Ser Leu Asn Ile Gln Ala Ser Gln Pro Ser Gly
    210                 215                 220

Leu Phe Lys Val Ser Lys Leu Ile Ser Asp Leu Asp Ser Thr Ala Val
225                 230                 235                 240

Tyr Ala Gln

<210>    13
<211>    235
<212>    PRT
<213>    Artificial Sequence

<220>
<223>    TuMV NIa ND8

<400>    13
Arg Asp Tyr Asn Pro Ile Ser Asn Asn Ile Cys His Leu Thr Asn Val
  1               5                  10                  15

Ser Asp Gly Ala Ser Asn Ser Leu Tyr Gly Val Gly Phe Gly Pro Leu
            20                  25                  30

Ile Leu Thr Asn Arg His Leu Phe Glu Arg Asn Asn Gly Glu Leu Val
        35                  40                  45

Ile Lys Ser Arg His Gly Glu Phe Val Ile Lys Asn Thr Thr Gln Leu
    50                  55                  60

His Leu Leu Pro Ile Pro Asp Arg Asp Leu Leu Leu Ile Arg Leu Pro
 65                  70                  75                  80

Lys Asp Ile Pro Pro Phe Pro Gln Lys Leu Gly Phe Arg Gln Pro Glu
                85                  90                  95

Lys Gly Glu Arg Ile Cys Met Val Gly Ser Asn Phe Gln Thr Lys Ser
            100                 105                 110

Ile Thr Ser Val Val Ser Glu Thr Ser Thr Ile Met Pro Val Glu Asn
        115                 120                 125

Ser Gln Phe Trp Lys His Trp Ile Ser Thr Lys Asp Gly Gln Cys Gly
    130                 135                 140

Ser Pro Met Val Ser Thr Lys Asp Gly Lys Ile Leu Gly Leu His Ser
145                 150                 155                 160

Leu Ala Asn Phe Gln Asn Ser Ile Asn Tyr Phe Ala Ala Phe Pro Asp
                165                 170                 175

Asp Phe Ala Glu Lys Tyr Leu His Thr Ile Glu Ala His Glu Trp Val
            180                 185                 190

Lys His Trp Lys Tyr Asn Thr Ser Ala Ile Ser Trp Gly Ser Leu Asn
        195                 200                 205

Ile Gln Ala Ser Gln Pro Ser Gly Leu Phe Lys Val Ser Lys Leu Ile
    210                 215                 220

Ser Asp Leu Asp Ser Thr Ala Val Tyr Ala Gln

225                 230                 235

<210>    14
<211>    215
<212>    PRT
<213>    Artificial Sequence

<220>
<223>    TuMV NIa ND8CD20

<400>    14
Arg Asp Tyr Asn Pro Ile Ser Asn Asn Ile Cys His Leu Thr Asn Val
1               5                   10                  15

Ser Asp Gly Ala Ser Asn Ser Leu Tyr Gly Val Gly Phe Gly Pro Leu
            20                  25                  30

Ile Leu Thr Asn Arg His Leu Phe Glu Arg Asn Asn Gly Glu Leu Val
        35                  40                  45

Ile Lys Ser Arg His Gly Glu Phe Val Ile Lys Asn Thr Thr Gln Leu
    50                  55                  60

His Leu Leu Pro Ile Pro Asp Arg Asp Leu Leu Leu Ile Arg Leu Pro
65                  70                  75                  80

Lys Asp Ile Pro Pro Phe Pro Gln Lys Leu Gly Phe Arg Gln Pro Glu
                85                  90                  95

Lys Gly Glu Arg Ile Cys Met Val Gly Ser Asn Phe Gln Thr Lys Ser
            100                 105                 110

Ile Thr Ser Val Val Ser Glu Thr Ser Thr Ile Met Pro Val Glu Asn
        115                 120                 125

Ser Gln Phe Trp Lys His Trp Ile Ser Thr Lys Asp Gly Gln Cys Gly
    130                 135                 140

Ser Pro Met Val Ser Thr Lys Asp Gly Lys Ile Leu Gly Leu His Ser
145                 150                 155                 160

Leu Ala Asn Phe Gln Asn Ser Ile Asn Tyr Phe Ala Ala Phe Pro Asp
                165                 170                 175

Asp Phe Ala Glu Lys Tyr Leu His Thr Ile Glu Ala His Glu Trp Val
            180                 185                 190

Lys His Trp Lys Tyr Asn Thr Ser Ala Ile Ser Trp Gly Ser Leu Asn
        195                 200                 205

Ile Gln Ala Ser Gln Pro Ser
    210                 215


<210>    15
<211>    214
<212>    PRT
<213>    Artificial Sequence

<220>
<223>    TuMV NIa ND8CD21

<400>    15
Arg Asp Tyr Asn Pro Ile Ser Asn Asn Ile Cys His Leu Thr Asn Val
1               5                   10                  15

Ser Asp Gly Ala Ser Asn Ser Leu Tyr Gly Val Gly Phe Gly Pro Leu
            20                  25                  30

Ile Leu Thr Asn Arg His Leu Phe Glu Arg Asn Asn Gly Glu Leu Val
        35                  40                  45

Ile Lys Ser Arg His Gly Glu Phe Val Ile Lys Asn Thr Thr Gln Leu
    50                  55                  60

His Leu Leu Pro Ile Pro Asp Arg Asp Leu Leu Leu Ile Arg Leu Pro
65                  70                  75                  80

Lys Asp Ile Pro Pro Phe Pro Gln Lys Leu Gly Phe Arg Gln Pro Glu
                85                  90                  95

Lys Gly Glu Arg Ile Cys Met Val Gly Ser Asn Phe Gln Thr Lys Ser
            100                 105                 110

Ile Thr Ser Val Val Ser Glu Thr Ser Thr Ile Met Pro Val Glu Asn
        115                 120                 125

Ser Gln Phe Trp Lys His Trp Ile Ser Thr Lys Asp Gly Gln Cys Gly
    130                 135                 140

Ser Pro Met Val Ser Thr Lys Asp Gly Lys Ile Leu Gly Leu His Ser
145                 150                 155                 160

Leu Ala Asn Phe Gln Asn Ser Ile Asn Tyr Phe Ala Ala Phe Pro Asp
                165                 170                 175

Asp Phe Ala Glu Lys Tyr Leu His Thr Ile Glu Ala His Glu Trp Val
            180                 185                 190

Lys His Trp Lys Tyr Asn Thr Ser Ala Ile Ser Trp Gly Ser Leu Asn
        195                 200                 205

Ile Gln Ala Ser Gln Pro
    210


<210>    16
<211>    211
<212>    PRT
<213>    Artificial Sequence

<220>
<223>    TuMV NIa ND8CD24


<400>    16
Arg Asp Tyr Asn Pro Ile Ser Asn Asn Ile Cys His Leu Thr Asn Val
1               5                   10                  15

Ser Asp Gly Ala Ser Asn Ser Leu Tyr Gly Val Gly Phe Gly Pro Leu
            20                  25                  30

Ile Leu Thr Asn Arg His Leu Phe Glu Arg Asn Asn Gly Glu Leu Val

35                  40                  45

Ile Lys Ser Arg His Gly Glu Phe Val Ile Lys Asn Thr Thr Gln Leu
    50                  55                  60

His Leu Leu Pro Ile Pro Asp Arg Asp Leu Leu Leu Ile Arg Leu Pro
65                  70                  75                  80

Lys Asp Ile Pro Pro Phe Pro Gln Lys Leu Gly Phe Arg Gln Pro Glu
                85                  90                  95

Lys Gly Glu Arg Ile Cys Met Val Gly Ser Asn Phe Gln Thr Lys Ser
            100                 105                 110

Ile Thr Ser Val Val Ser Glu Thr Ser Thr Ile Met Pro Val Glu Asn
        115                 120                 125

Ser Gln Phe Trp Lys His Trp Ile Ser Thr Lys Asp Gly Gln Cys Gly
    130                 135                 140

Ser Pro Met Val Ser Thr Lys Asp Gly Lys Ile Leu Gly Leu His Ser
145                 150                 155                 160

Leu Ala Asn Phe Gln Asn Ser Ile Asn Tyr Phe Ala Ala Phe Pro Asp
                165                 170                 175

Asp Phe Ala Glu Lys Tyr Leu His Thr Ile Glu Ala His Glu Trp Val
            180                 185                 190

Lys His Trp Lys Tyr Asn Thr Ser Ala Ile Ser Trp Gly Ser Leu Asn
        195                 200                 205

Ile Gln Ala
    210


<210>    17
<211>    207
<212>    PRT
<213>    Artificial Sequence

<220>
<223>    TuMV NIa ND8CD28


<400>    17
Arg Asp Tyr Asn Pro Ile Ser Asn Asn Ile Cys His Leu Thr Asn Val
1               5                   10                  15

Ser Asp Gly Ala Ser Asn Ser Leu Tyr Gly Val Gly Phe Gly Pro Leu
            20                  25                  30

Ile Leu Thr Asn Arg His Leu Phe Glu Arg Asn Asn Gly Glu Leu Val
        35                  40                  45

Ile Lys Ser Arg His Gly Glu Phe Val Ile Lys Asn Thr Thr Gln Leu
    50                  55                  60

His Leu Leu Pro Ile Pro Asp Arg Asp Leu Leu Leu Ile Arg Leu Pro
65                  70                  75                  80

Lys Asp Ile Pro Pro Phe Pro Gln Lys Leu Gly Phe Arg Gln Pro Glu
                85                  90                  95

Lys Gly Glu Arg Ile Cys Met Val Gly Ser Asn Phe Gln Thr Lys Ser
            100                 105                 110

Ile Thr Ser Val Val Ser Glu Thr Ser Thr Ile Met Pro Val Glu Asn
        115                 120                 125

Ser Gln Phe Trp Lys His Trp Ile Ser Thr Lys Asp Gly Gln Cys Gly
    130                 135                 140

Ser Pro Met Val Ser Thr Lys Asp Gly Lys Ile Leu Gly Leu His Ser
145                 150                 155                 160

Leu Ala Asn Phe Gln Asn Ser Ile Asn Tyr Phe Ala Ala Phe Pro Asp
                165                 170                 175

Asp Phe Ala Glu Lys Tyr Leu His Thr Ile Glu Ala His Glu Trp Val
            180                 185                 190

Lys His Trp Lys Tyr Asn Thr Ser Ala Ile Ser Trp Gly Ser Leu
        195                 200                 205

<210> 18
<211> 214
<212> PRT
<213> Artificial Sequence

<220>
<223> TuMV NIa ND8CD21(D73N)

<400> 18
Arg Asp Tyr Asn Pro Ile Ser Asn Asn Ile Cys His Leu Thr Asn Val
1               5                   10                  15

Ser Asp Gly Ala Ser Asn Ser Leu Tyr Gly Val Gly Phe Gly Pro Leu
            20                  25                  30

Ile Leu Thr Asn Arg His Leu Phe Glu Arg Asn Asn Gly Glu Leu Val
        35                  40                  45

Ile Lys Ser Arg His Gly Glu Phe Val Ile Lys Asn Thr Thr Gln Leu
    50                  55                  60

His Leu Leu Pro Ile Pro Asp Arg Asn Leu Leu Leu Ile Arg Leu Pro
65                  70                  75                  80

Lys Asp Ile Pro Pro Phe Pro Gln Lys Leu Gly Phe Arg Gln Pro Glu
                85                  90                  95

Lys Gly Glu Arg Ile Cys Met Val Gly Ser Asn Phe Gln Thr Lys Ser
            100                 105                 110

Ile Thr Ser Val Val Ser Glu Thr Ser Thr Ile Met Pro Val Glu Asn
        115                 120                 125

Ser Gln Phe Trp Lys His Trp Ile Ser Thr Lys Asp Gly Gln Cys Gly
    130                 135                 140

Ser Pro Met Val Ser Thr Lys Asp Gly Lys Ile Leu Gly Leu His Ser

145                 150                 155                 160

Leu Ala Asn Phe Gln Asn Ser Ile Asn Tyr Phe Ala Ala Phe Pro Asp
                165                 170                 175

Asp Phe Ala Glu Lys Tyr Leu His Thr Ile Glu Ala His Glu Trp Val
            180                 185                 190

Lys His Trp Lys Tyr Asn Thr Ser Ala Ile Ser Trp Gly Ser Leu Asn
        195                 200                 205

Ile Gln Ala Ser Gln Pro
    210

<210>    19
<211>    214
<212>    PRT
<213>    Artificial Sequence

<220>
<223>    TuMV NIa ND8CD21(N15Q)

<400>    19
Arg Asp Tyr Asn Pro Ile Ser Asn Asn Ile Cys His Leu Thr Gln Val
 1               5                  10                  15

Ser Asp Gly Ala Ser Asn Ser Leu Tyr Gly Val Gly Phe Gly Pro Leu
            20                  25                  30

Ile Leu Thr Asn Arg His Leu Phe Glu Arg Asn Asn Gly Glu Leu Val
        35                  40                  45

Ile Lys Ser Arg His Gly Glu Phe Val Ile Lys Asn Thr Thr Gln Leu
    50                  55                  60

His Leu Leu Pro Ile Pro Asp Arg Asp Leu Leu Leu Ile Arg Leu Pro
65                  70                  75                  80

Lys Asp Ile Pro Pro Phe Pro Gln Lys Leu Gly Phe Arg Gln Pro Glu
                85                  90                  95

Lys Gly Glu Arg Ile Cys Met Val Gly Ser Asn Phe Gln Thr Lys Ser
            100                 105                 110

Ile Thr Ser Val Val Ser Glu Thr Ser Thr Ile Met Pro Val Glu Asn
        115                 120                 125

Ser Gln Phe Trp Lys His Trp Ile Ser Thr Lys Asp Gly Gln Cys Gly
    130                 135                 140

Ser Pro Met Val Ser Thr Lys Asp Gly Lys Ile Leu Gly Leu His Ser
145                 150                 155                 160

Leu Ala Asn Phe Gln Asn Ser Ile Asn Tyr Phe Ala Ala Phe Pro Asp
                165                 170                 175

Asp Phe Ala Glu Lys Tyr Leu His Thr Ile Glu Ala His Glu Trp Val
            180                 185                 190

Lys His Trp Lys Tyr Asn Thr Ser Ala Ile Ser Trp Gly Ser Leu Asn
        195                 200                 205

Ile Gln Ala Ser Gln Pro
    210

<210>    20
<211>    214
<212>    PRT
<213>    Artificial Sequence

<220>
<223>    TuMV NIa ND8CD21(N60Q)

<400>    20
Arg Asp Tyr Asn Pro Ile Ser Asn Asn Ile Cys His Leu Thr Asn Val
1               5                   10                  15

Ser Asp Gly Ala Ser Asn Ser Leu Tyr Gly Val Gly Phe Gly Pro Leu
            20                  25                  30

Ile Leu Thr Asn Arg His Leu Phe Glu Arg Asn Asn Gly Glu Leu Val
        35                  40                  45

Ile Lys Ser Arg His Gly Glu Phe Val Ile Lys Gln Thr Thr Gln Leu
    50                  55                  60

His Leu Leu Pro Ile Pro Asp Arg Asp Leu Leu Leu Ile Arg Leu Pro
65                  70                  75                  80

Lys Asp Ile Pro Pro Phe Pro Gln Lys Leu Gly Phe Arg Gln Pro Glu
                85                  90                  95

Lys Gly Glu Arg Ile Cys Met Val Gly Ser Asn Phe Gln Thr Lys Ser
            100                 105                 110

Ile Thr Ser Val Val Ser Glu Thr Ser Thr Ile Met Pro Val Glu Asn
        115                 120                 125

Ser Gln Phe Trp Lys His Trp Ile Ser Thr Lys Asp Gly Gln Cys Gly
    130                 135                 140

Ser Pro Met Val Ser Thr Lys Asp Gly Lys Ile Leu Gly Leu His Ser
145                 150                 155                 160

Leu Ala Asn Phe Gln Asn Ser Ile Asn Tyr Phe Ala Ala Phe Pro Asp
                165                 170                 175

Asp Phe Ala Glu Lys Tyr Leu His Thr Ile Glu Ala His Glu Trp Val
            180                 185                 190

Lys His Trp Lys Tyr Asn Thr Ser Ala Ile Ser Trp Gly Ser Leu Asn
        195                 200                 205

Ile Gln Ala Ser Gln Pro
    210

<210>    21
<211>    214
<212>    PRT
<213>    Artificial Sequence

<220>
<223>    TuMV NIa ND8CD21(N198Q)

<400>    21
Arg Asp Tyr Asn Pro Ile Ser Asn Asn Ile Cys His Leu Thr Asn Val
1               5                   10                  15

Ser Asp Gly Ala Ser Asn Ser Leu Tyr Gly Val Gly Phe Gly Pro Leu
            20                  25                  30

Ile Leu Thr Asn Arg His Leu Phe Glu Arg Asn Asn Gly Glu Leu Val
        35                  40                  45

Ile Lys Ser Arg His Gly Glu Phe Val Ile Lys Asn Thr Thr Gln Leu
    50                  55                  60

His Leu Leu Pro Ile Pro Asp Arg Asp Leu Leu Leu Ile Arg Leu Pro
65                  70                  75                  80

Lys Asp Ile Pro Pro Phe Pro Gln Lys Leu Gly Phe Arg Gln Pro Glu
                85                  90                  95

Lys Gly Glu Arg Ile Cys Met Val Gly Ser Asn Phe Gln Thr Lys Ser
            100                 105                 110

Ile Thr Ser Val Val Ser Glu Thr Ser Thr Ile Met Pro Val Glu Asn
        115                 120                 125

Ser Gln Phe Trp Lys His Trp Ile Ser Thr Lys Asp Gly Gln Cys Gly
    130                 135                 140

Ser Pro Met Val Ser Thr Lys Asp Gly Lys Ile Leu Gly Leu His Ser
145                 150                 155                 160

Leu Ala Asn Phe Gln Asn Ser Ile Asn Tyr Phe Ala Ala Phe Pro Asp
                165                 170                 175

Asp Phe Ala Glu Lys Tyr Leu His Thr Ile Glu Ala His Glu Trp Val
            180                 185                 190

Lys His Trp Lys Tyr Gln Thr Ser Ala Ile Ser Trp Gly Ser Leu Asn
        195                 200                 205

Ile Gln Ala Ser Gln Pro
    210

<210>    22
<211>    9
<212>    PRT
<213>    Artificial Sequence

<220>
<223>    L2

<400>    22
Gln Thr Asp Glu Ser Thr Leu Met Thr
1               5

<210>    23

<211>    8
<212>    PRT
<213>    Artificial Sequence

<220>
<223>    L2


<400>    23
Thr Asp Glu Ser Thr Leu Met Thr
1               5


<210>    24
<211>    8
<212>    PRT
<213>    Artificial Sequence

<220>
<223>    L2


<400>    24
Gln Thr Asp Glu Ser Thr Leu Met
1               5


<210>    25
<211>    7
<212>    PRT
<213>    Artificial Sequence

<220>
<223>    L2


<400>    25
Asp Glu Ser Thr Leu Met Thr
1               5


<210>    26
<211>    7
<212>    PRT
<213>    Artificial Sequence

<220>
<223>    L2


<400>    26
Gln Thr Asp Glu Ser Thr Leu
1               5


<210>    27
<211>    6
<212>    PRT
<213>    Artificial Sequence

<220>
<223>    L2

<400>    27
Glu Ser Thr Leu Met Thr
1               5


<210>    28
<211>    6
<212>    PRT
<213>    Artificial Sequence

<220>
<223>    L2


<400>    28
Gln Thr Asp Glu Ser Thr
1               5


<210>    29
<211>    21
<212>    PRT
<213>    Artificial Sequence

<220>
<223>    Transmembrane domain


<400>    29
Ile Ala Tyr Val Met Ala Ala Ile Cys Ala Leu Phe Met Leu Pro Leu
1               5                   10                  15

Cys Leu Met Val Cys
            20


<210>    30
<211>    23
<212>    PRT
<213>    Artificial Sequence

<220>
<223>    Intracellular domain


<400>    30
Gln Trp Arg Cys Leu Arg Cys Leu Arg Gln Gln His Asp Asp Phe Ala
1               5                   10                  15

Asp Asp Ile Ser Leu Leu Lys
            20


<210>    31
<211>    335
<212>    PRT
<213>    Artificial Sequence

<220>
<223>    SAS

<400> 31
Met Ala Gln Ala Leu Pro Trp Leu Leu Leu Trp Met Gly Ala Gly Val
1               5                   10                  15

Leu Pro Ala His Gly Thr Gln His Gly Ile Arg Leu Pro Leu Arg Ser
            20                  25                  30

Gly Leu Gly Gly Ala Pro Leu Gly Leu Arg Leu Pro Arg Glu Thr Asp
        35                  40                  45

Glu Glu Pro Glu Glu Pro Gly Arg Asp Tyr Asn Pro Ile Ser Asn Asn
    50                  55                  60

Ile Cys His Leu Thr Asn Val Ser Asp Gly Ala Ser Asn Ser Leu Tyr
65                  70                  75                  80

Gly Val Gly Phe Gly Pro Leu Ile Leu Thr Asn Arg His Leu Phe Glu
                85                  90                  95

Arg Asn Asn Gly Glu Leu Val Ile Lys Ser Arg His Gly Glu Phe Val
            100                 105                 110

Ile Lys Asn Thr Thr Gln Leu His Leu Leu Pro Ile Pro Asp Arg Asp
        115                 120                 125

Leu Leu Leu Ile Arg Leu Pro Lys Asp Ile Pro Pro Phe Pro Gln Lys
    130                 135                 140

Leu Gly Phe Arg Gln Pro Glu Lys Gly Glu Arg Ile Cys Met Val Gly
145                 150                 155                 160

Ser Asn Phe Gln Thr Lys Ser Ile Thr Ser Val Val Ser Glu Thr Ser
                165                 170                 175

Thr Ile Met Pro Val Glu Asn Ser Gln Phe Trp Lys His Trp Ile Ser
            180                 185                 190

Thr Lys Asp Gly Gln Cys Gly Ser Pro Met Val Ser Thr Lys Asp Gly
        195                 200                 205

Lys Ile Leu Gly Leu His Ser Leu Ala Asn Phe Gln Asn Ser Ile Asn
    210                 215                 220

Tyr Phe Ala Ala Phe Pro Asp Asp Phe Ala Glu Lys Tyr Leu His Thr
225                 230                 235                 240

Ile Glu Ala His Glu Trp Val Lys His Trp Lys Tyr Asn Thr Ser Ala
                245                 250                 255

Ile Ser Trp Gly Ser Leu Asn Ile Gln Ala Ser Gln Pro Asn Ile Pro
            260                 265                 270

Gln Thr Asp Glu Ser Thr Leu Met Thr Ile Ala Tyr Val Met Ala Ala
        275                 280                 285

Ile Cys Ala Leu Phe Met Leu Pro Leu Cys Leu Met Val Cys Gln Trp
    290                 295                 300

Arg Cys Leu Arg Cys Leu Arg Gln Gln His Asp Asp Phe Ala Asp Asp
305                 310                 315                 320

Ile Ser Leu Leu Lys Glu Gln Lys Leu Ile Ser Glu Glu Asp Leu
                325                 330                 335

<210>    32
<211>    335
<212>    PRT
<213>    Artificial Sequence

<220>
<223>    SAS(N70Q)


<400>    32
Met Ala Gln Ala Leu Pro Trp Leu Leu Leu Trp Met Gly Ala Gly Val
1               5                   10                  15

Leu Pro Ala His Gly Thr Gln His Gly Ile Arg Leu Pro Leu Arg Ser
            20                  25                  30

Gly Leu Gly Gly Ala Pro Leu Gly Leu Arg Leu Pro Arg Glu Thr Asp
        35                  40                  45

Glu Glu Pro Glu Glu Pro Gly Arg Asp Tyr Asn Pro Ile Ser Asn Asn
    50                  55                  60

Ile Cys His Leu Thr Gln Val Ser Asp Gly Ala Ser Asn Ser Leu Tyr
65                  70                  75                  80

Gly Val Gly Phe Gly Pro Leu Ile Leu Thr Asn Arg His Leu Phe Glu
                85                  90                  95

Arg Asn Asn Gly Glu Leu Val Ile Lys Ser Arg His Gly Glu Phe Val
            100                 105                 110

Ile Lys Asn Thr Thr Gln Leu His Leu Leu Pro Ile Pro Asp Arg Asp
        115                 120                 125

Leu Leu Leu Ile Arg Leu Pro Lys Asp Ile Pro Pro Phe Pro Gln Lys
    130                 135                 140

Leu Gly Phe Arg Gln Pro Glu Lys Gly Glu Arg Ile Cys Met Val Gly
145                 150                 155                 160

Ser Asn Phe Gln Thr Lys Ser Ile Thr Ser Val Val Ser Glu Thr Ser
                165                 170                 175

Thr Ile Met Pro Val Glu Asn Ser Gln Phe Trp Lys His Trp Ile Ser
            180                 185                 190

Thr Lys Asp Gly Gln Cys Gly Ser Pro Met Val Ser Thr Lys Asp Gly
        195                 200                 205

Lys Ile Leu Gly Leu His Ser Leu Ala Asn Phe Gln Asn Ser Ile Asn
    210                 215                 220

Tyr Phe Ala Ala Phe Pro Asp Asp Phe Ala Glu Lys Tyr Leu His Thr
225                 230                 235                 240

Ile Glu Ala His Glu Trp Val Lys His Trp Lys Tyr Asn Thr Ser Ala
                245                 250                 255

Ile Ser Trp Gly Ser Leu Asn Ile Gln Ala Ser Gln Pro Asn Ile Pro
            260                 265                 270

Gln Thr Asp Glu Ser Thr Leu Met Thr Ile Ala Tyr Val Met Ala Ala
        275                 280                 285

Ile Cys Ala Leu Phe Met Leu Pro Leu Cys Leu Met Val Cys Gln Trp
    290                 295                 300

Arg Cys Leu Arg Cys Leu Arg Gln Gln His Asp Asp Phe Ala Asp Asp
305                 310                 315                 320

Ile Ser Leu Leu Lys Glu Gln Lys Leu Ile Ser Glu Glu Asp Leu
                325                 330                 335

<210>    33
<211>    335
<212>    PRT
<213>    Artificial Sequence

<220>
<223>    SAS(N115Q)

<400>    33
Met Ala Gln Ala Leu Pro Trp Leu Leu Leu Trp Met Gly Ala Gly Val
  1               5                  10                  15

Leu Pro Ala His Gly Thr Gln His Gly Ile Arg Leu Pro Leu Arg Ser
             20                  25                  30

Gly Leu Gly Gly Ala Pro Leu Gly Leu Arg Leu Pro Arg Glu Thr Asp
         35                  40                  45

Glu Glu Pro Glu Glu Pro Gly Arg Asp Tyr Asn Pro Ile Ser Asn Asn
     50                  55                  60

Ile Cys His Leu Thr Asn Val Ser Asp Gly Ala Ser Asn Ser Leu Tyr
 65                  70                  75                  80

Gly Val Gly Phe Gly Pro Leu Ile Leu Thr Asn Arg His Leu Phe Glu
                 85                  90                  95

Arg Asn Asn Gly Glu Leu Val Ile Lys Ser Arg His Gly Glu Phe Val
            100                 105                 110

Ile Lys Gln Thr Thr Gln Leu His Leu Leu Pro Ile Pro Asp Arg Asp
        115                 120                 125

Leu Leu Leu Ile Arg Leu Pro Lys Asp Ile Pro Pro Phe Pro Gln Lys
    130                 135                 140

Leu Gly Phe Arg Gln Pro Glu Lys Gly Glu Arg Ile Cys Met Val Gly
145                 150                 155                 160

Ser Asn Phe Gln Thr Lys Ser Ile Thr Ser Val Val Ser Glu Thr Ser
                165                 170                 175

Thr Ile Met Pro Val Glu Asn Ser Gln Phe Trp Lys His Trp Ile Ser
            180                 185                 190

Thr Lys Asp Gly Gln Cys Gly Ser Pro Met Val Ser Thr Lys Asp Gly
        195                 200                 205

Lys Ile Leu Gly Leu His Ser Leu Ala Asn Phe Gln Asn Ser Ile Asn

210                 215                 220

Tyr Phe Ala Ala Phe Pro Asp Asp Phe Ala Glu Lys Tyr Leu His Thr
225                 230                 235                 240

Ile Glu Ala His Glu Trp Val Lys His Trp Lys Tyr Asn Thr Ser Ala
                245                 250                 255

Ile Ser Trp Gly Ser Leu Asn Ile Gln Ala Ser Gln Pro Asn Ile Pro
            260                 265                 270

Gln Thr Asp Glu Ser Thr Leu Met Thr Ile Ala Tyr Val Met Ala Ala
        275                 280                 285

Ile Cys Ala Leu Phe Met Leu Pro Leu Cys Leu Met Val Cys Gln Trp
    290                 295                 300

Arg Cys Leu Arg Cys Leu Arg Gln Gln His Asp Asp Phe Ala Asp Asp
305                 310                 315                 320

Ile Ser Leu Leu Lys Glu Gln Lys Leu Ile Ser Glu Glu Asp Leu
                325                 330                 335


<210>    34
<211>    335
<212>    PRT
<213>    Artificial Sequence

<220>
<223>    SAS(N253Q)


<400>    34
Met Ala Gln Ala Leu Pro Trp Leu Leu Leu Trp Met Gly Ala Gly Val
1               5                   10                  15

Leu Pro Ala His Gly Thr Gln His Gly Ile Arg Leu Pro Leu Arg Ser
            20                  25                  30

Gly Leu Gly Gly Ala Pro Leu Gly Leu Arg Leu Pro Arg Glu Thr Asp
        35                  40                  45

Glu Glu Pro Glu Glu Pro Gly Arg Asp Tyr Asn Pro Ile Ser Asn Asn
    50                  55                  60

Ile Cys His Leu Thr Asn Val Ser Asp Gly Ala Ser Asn Ser Leu Tyr
65                  70                  75                  80

Gly Val Gly Phe Gly Pro Leu Ile Leu Thr Asn Arg His Leu Phe Glu
                85                  90                  95

Arg Asn Asn Gly Glu Leu Val Ile Lys Ser Arg His Gly Glu Phe Val
            100                 105                 110

Ile Lys Asn Thr Thr Gln Leu His Leu Leu Pro Ile Pro Asp Arg Asp
        115                 120                 125

Leu Leu Leu Ile Arg Leu Pro Lys Asp Ile Pro Pro Phe Pro Gln Lys
    130                 135                 140

Leu Gly Phe Arg Gln Pro Glu Lys Gly Glu Arg Ile Cys Met Val Gly
145                 150                 155                 160

Ser Asn Phe Gln Thr Lys Ser Ile Thr Ser Val Val Ser Glu Thr Ser
                165                 170                 175

Thr Ile Met Pro Val Glu Asn Ser Gln Phe Trp Lys His Trp Ile Ser
            180                 185                 190

Thr Lys Asp Gly Gln Cys Gly Ser Pro Met Val Ser Thr Lys Asp Gly
        195                 200                 205

Lys Ile Leu Gly Leu His Ser Leu Ala Asn Phe Gln Asn Ser Ile Asn
    210                 215                 220

Tyr Phe Ala Ala Phe Pro Asp Asp Phe Ala Glu Lys Tyr Leu His Thr
225                 230                 235                 240

Ile Glu Ala His Glu Trp Val Lys His Trp Lys Tyr Gln Thr Ser Ala
                245                 250                 255

Ile Ser Trp Gly Ser Leu Asn Ile Gln Ala Ser Gln Pro Asn Ile Pro
            260                 265                 270

Gln Thr Asp Glu Ser Thr Leu Met Thr Ile Ala Tyr Val Met Ala Ala
        275                 280                 285

Ile Cys Ala Leu Phe Met Leu Pro Leu Cys Leu Met Val Cys Gln Trp
    290                 295                 300

Arg Cys Leu Arg Cys Leu Arg Gln Gln His Asp Asp Phe Ala Asp Asp
305                 310                 315                 320

Ile Ser Leu Leu Lys Glu Gln Lys Leu Ile Ser Glu Glu Asp Leu
                325                 330                 335


<210>    35
<211>    335
<212>    PRT
<213>    Artificial Sequence

<220>
<223>    SAS(m)


<400>    35
Met Ala Gln Ala Leu Pro Trp Leu Leu Leu Trp Met Gly Ala Gly Val
 1               5                  10                  15

Leu Pro Ala His Gly Thr Gln His Gly Ile Arg Leu Pro Leu Arg Ser
            20                  25                  30

Gly Leu Gly Gly Ala Pro Leu Gly Leu Arg Leu Pro Arg Glu Thr Asp
        35                  40                  45

Glu Glu Pro Glu Glu Pro Gly Arg Asp Tyr Asn Pro Ile Ser Asn Asn
    50                  55                  60

Ile Cys His Leu Thr Asn Val Ser Asp Gly Ala Ser Asn Ser Leu Tyr
 65                 70                  75                  80

Gly Val Gly Phe Gly Pro Leu Ile Leu Thr Asn Arg His Leu Phe Glu
                85                  90                  95

Arg Asn Asn Gly Glu Leu Val Ile Lys Ser Arg His Gly Glu Phe Val
            100                 105                 110

Ile Lys Asn Thr Thr Gln Leu His Leu Leu Pro Ile Pro Asp Arg Asn
        115                 120                 125

Leu Leu Leu Ile Arg Leu Pro Lys Asp Ile Pro Pro Phe Pro Gln Lys
    130                 135                 140

Leu Gly Phe Arg Gln Pro Glu Lys Gly Glu Arg Ile Cys Met Val Gly
145                 150                 155                 160

Ser Asn Phe Gln Thr Lys Ser Ile Thr Ser Val Val Ser Glu Thr Ser
                165                 170                 175

Thr Ile Met Pro Val Glu Asn Ser Gln Phe Trp Lys His Trp Ile Ser
            180                 185                 190

Thr Lys Asp Gly Gln Cys Gly Ser Pro Met Val Ser Thr Lys Asp Gly
        195                 200                 205

Lys Ile Leu Gly Leu His Ser Leu Ala Asn Phe Gln Asn Ser Ile Asn
    210                 215                 220

Tyr Phe Ala Ala Phe Pro Asp Asp Phe Ala Glu Lys Tyr Leu His Thr
225                 230                 235                 240

Ile Glu Ala His Glu Trp Val Lys His Trp Lys Tyr Asn Thr Ser Ala
                245                 250                 255

Ile Ser Trp Gly Ser Leu Asn Ile Gln Ala Ser Gln Pro Asn Ile Pro
            260                 265                 270

Gln Thr Asp Glu Ser Thr Leu Met Thr Ile Ala Tyr Val Met Ala Ala
        275                 280                 285

Ile Cys Ala Leu Phe Met Leu Pro Leu Cys Leu Met Val Cys Gln Trp
    290                 295                 300

Arg Cys Leu Arg Cys Leu Arg Gln Gln His Asp Asp Phe Ala Asp Asp
305                 310                 315                 320

Ile Ser Leu Leu Lys Glu Gln Lys Leu Ile Ser Glu Glu Asp Leu
                325                 330                 335


<210>   36
<211>   315
<212>   PRT
<213>   Artificial Sequence

<220>
<223>   SAS(d20)


<400>   36
Met Ala Gln Ala Leu Pro Trp Leu Leu Leu Trp Met Gly Ala Gly Val
1               5                   10                  15

Leu Pro Ala His Gly Thr Gln His Gly Ile Arg Leu Pro Leu Arg Ser
            20                  25                  30

Gly Leu Gly Gly Ala Pro Leu Gly Leu Arg Leu Pro Arg Glu Thr Asp

        35                  40                  45

Glu Glu Pro Glu Glu Pro Gly Arg Asp Tyr Asn Pro Ile Ser Asn Asn
    50                  55                  60

Ile Cys His Leu Thr Asn Val Ser Asp Gly Ala Ser Asn Ser Leu Tyr
65                  70                  75                  80

Gly Val Gly Phe Gly Pro Leu Ile Leu Thr Asn Arg His Leu Phe Glu
                85                  90                  95

Arg Asn Asn Gly Glu Leu Val Ile Lys Ser Arg His Gly Glu Phe Val
            100                 105                 110

Ile Lys Asn Thr Thr Gln Leu His Leu Leu Pro Ile Pro Asp Arg Asp
        115                 120                 125

Leu Leu Leu Ile Arg Leu Pro Lys Asp Ile Pro Pro Phe Pro Gln Lys
    130                 135                 140

Leu Gly Phe Arg Gln Pro Glu Lys Gly Glu Arg Ile Cys Met Val Gly
145                 150                 155                 160

Ser Asn Phe Gln Thr Lys Ser Ile Thr Ser Val Val Ser Glu Thr Ser
                165                 170                 175

Thr Ile Met Pro Val Glu Asn Ser Gln Phe Trp Lys His Trp Ile Ser
            180                 185                 190

Thr Lys Asp Gly Gln Cys Gly Ser Pro Met Val Ser Thr Lys Asp Gly
        195                 200                 205

Lys Ile Leu Gly Leu His Ser Leu Ala Asn Phe Gln Asn Ser Ile Asn
    210                 215                 220

Tyr Phe Ala Ala Phe Pro Asp Asp Phe Ala Glu Lys Tyr Leu His Thr
225                 230                 235                 240

Ile Glu Ala His Glu Trp Val Lys His Trp Lys Tyr Asn Thr Ser Ala
                245                 250                 255

Ile Ser Trp Gly Ser Leu Asn Ile Gln Ala Ser Gln Pro Asn Ile Pro
            260                 265                 270

Gln Thr Asp Glu Ser Thr Leu Met Thr Ile Ala Tyr Val Met Ala Ala
        275                 280                 285

Ile Cys Ala Leu Phe Met Leu Pro Leu Cys Leu Met Val Cys Gln Trp
    290                 295                 300

Arg Glu Gln Lys Leu Ile Ser Glu Glu Asp Leu
305                 310                 315

<210> 37
<211> 280
<212> PRT
<213> Artificial Sequence

<220>
<223> SAS(SEC)

<400>    37
Met Ala Gln Ala Leu Pro Trp Leu Leu Leu Trp Met Gly Ala Gly Val
1               5                   10                  15

Leu Pro Ala His Gly Thr Gln His Gly Ile Arg Leu Pro Leu Arg Ser
            20                  25                  30

Gly Leu Gly Gly Ala Pro Leu Gly Leu Arg Leu Pro Arg Glu Thr Asp
        35                  40                  45

Glu Glu Pro Glu Glu Pro Gly Arg Asp Tyr Asn Pro Ile Ser Asn Asn
    50                  55                  60

Ile Cys His Leu Thr Asn Val Ser Asp Gly Ala Ser Asn Ser Leu Tyr
65                  70                  75                  80

Gly Val Gly Phe Gly Pro Leu Ile Leu Thr Asn Arg His Leu Phe Glu
                85                  90                  95

Arg Asn Asn Gly Glu Leu Val Ile Lys Ser Arg His Gly Glu Phe Val
            100                 105                 110

Ile Lys Asn Thr Thr Gln Leu His Leu Leu Pro Ile Pro Asp Arg Asp
        115                 120                 125

Leu Leu Leu Ile Arg Leu Pro Lys Asp Ile Pro Pro Phe Pro Gln Lys
    130                 135                 140

Leu Gly Phe Arg Gln Pro Glu Lys Gly Glu Arg Ile Cys Met Val Gly
145                 150                 155                 160

Ser Asn Phe Gln Thr Lys Ser Ile Thr Ser Val Val Ser Glu Thr Ser
                165                 170                 175

Thr Ile Met Pro Val Glu Asn Ser Gln Phe Trp Lys His Trp Ile Ser
            180                 185                 190

Thr Lys Asp Gly Gln Cys Gly Ser Pro Met Val Ser Thr Lys Asp Gly
        195                 200                 205

Lys Ile Leu Gly Leu His Ser Leu Ala Asn Phe Gln Asn Ser Ile Asn
    210                 215                 220

Tyr Phe Ala Ala Phe Pro Asp Asp Phe Ala Glu Lys Tyr Leu His Thr
225                 230                 235                 240

Ile Glu Ala His Glu Trp Val Lys His Trp Lys Tyr Asn Thr Ser Ala
                245                 250                 255

Ile Ser Trp Gly Ser Leu Asn Ile Gln Ala Ser Gln Pro Asn Glu Gln
            260                 265                 270

Lys Leu Ile Ser Glu Glu Asp Leu
        275                 280


<210>    38
<211>    303
<212>    PRT
<213>    Artificial Sequence

<220>
<223>    SAS(GPI)

<400>     38
Met Ala Gln Ala Leu Pro Trp Leu Leu Leu Trp Met Gly Ala Gly Val
1               5                   10                  15

Leu Pro Ala His Gly Thr Gln His Gly Ile Arg Leu Pro Leu Arg Ser
            20                  25                  30

Gly Leu Gly Gly Ala Pro Leu Gly Leu Arg Leu Pro Arg Glu Thr Asp
        35                  40                  45

Glu Glu Pro Glu Glu Pro Gly Arg Asp Tyr Asn Pro Ile Ser Asn Asn
    50                  55                  60

Ile Cys His Leu Thr Asn Val Ser Asp Gly Ala Ser Asn Ser Leu Tyr
65                  70                  75                  80

Gly Val Gly Phe Gly Pro Leu Ile Leu Thr Asn Arg His Leu Phe Glu
                85                  90                  95

Arg Asn Asn Gly Glu Leu Val Ile Lys Ser Arg His Gly Glu Phe Val
            100                 105                 110

Ile Lys Asn Thr Thr Gln Leu His Leu Leu Pro Ile Pro Asp Arg Asp
        115                 120                 125

Leu Leu Leu Ile Arg Leu Pro Lys Asp Ile Pro Pro Phe Pro Gln Lys
    130                 135                 140

Leu Gly Phe Arg Gln Pro Glu Lys Gly Glu Arg Ile Cys Met Val Gly
145                 150                 155                 160

Ser Asn Phe Gln Thr Lys Ser Ile Thr Ser Val Val Ser Glu Thr Ser
                165                 170                 175

Thr Ile Met Pro Val Glu Asn Ser Gln Phe Trp Lys His Trp Ile Ser
            180                 185                 190

Thr Lys Asp Gly Gln Cys Gly Ser Pro Met Val Ser Thr Lys Asp Gly
        195                 200                 205

Lys Ile Leu Gly Leu His Ser Leu Ala Asn Phe Gln Asn Ser Ile Asn
    210                 215                 220

Tyr Phe Ala Ala Phe Pro Asp Asp Phe Ala Glu Lys Tyr Leu His Thr
225                 230                 235                 240

Ile Glu Ala His Glu Trp Val Lys His Trp Lys Tyr Asn Thr Ser Ala
                245                 250                 255

Ile Ser Trp Gly Ser Leu Asn Ile Gln Ala Ser Gln Pro Glu Gln Lys
            260                 265                 270

Leu Ile Ser Glu Glu Asp Leu Pro Asp His Ser Ala Ala Thr Lys Pro
        275                 280                 285

Ser Leu Phe Leu Phe Leu Val Ser Leu Leu His Ile Phe Phe Lys
    290                 295                 300

<210>    39
<211>    306
<212>    PRT
<213>    Artificial Sequence

<220>
<223>    SAS2


<400>    39
Met Ala Gln Ala Leu Pro Trp Leu Leu Leu Trp Met Gly Ala Gly Val
1               5                   10                  15

Leu Pro Ala His Gly Thr Gln His Gly Ile Arg Asp Tyr Asn Pro Ile
            20                  25                  30

Ser Asn Asn Ile Cys His Leu Thr Asn Val Ser Asp Gly Ala Ser Asn
        35                  40                  45

Ser Leu Tyr Gly Val Gly Phe Gly Pro Leu Ile Leu Thr Asn Arg His
    50                  55                  60

Leu Phe Glu Arg Asn Asn Gly Glu Leu Val Ile Lys Ser Arg His Gly
65                  70                  75                  80

Glu Phe Val Ile Lys Asn Thr Thr Gln Leu His Leu Leu Pro Ile Pro
                85                  90                  95

Asp Arg Asp Leu Leu Leu Ile Arg Leu Pro Lys Asp Ile Pro Pro Phe
            100                 105                 110

Pro Gln Lys Leu Gly Phe Arg Gln Pro Glu Lys Gly Glu Arg Ile Cys
        115                 120                 125

Met Val Gly Ser Asn Phe Gln Thr Lys Ser Ile Thr Ser Val Val Ser
    130                 135                 140

Glu Thr Ser Thr Ile Met Pro Val Glu Asn Ser Gln Phe Trp Lys His
145                 150                 155                 160

Trp Ile Ser Thr Lys Asp Gly Gln Cys Gly Ser Pro Met Val Ser Thr
                165                 170                 175

Lys Asp Gly Lys Ile Leu Gly Leu His Ser Leu Ala Asn Phe Gln Asn
            180                 185                 190

Ser Ile Asn Tyr Phe Ala Ala Phe Pro Asp Asp Phe Ala Glu Lys Tyr
        195                 200                 205

Leu His Thr Ile Glu Ala His Glu Trp Val Lys His Trp Lys Tyr Asn
    210                 215                 220

Thr Ser Ala Ile Ser Trp Gly Ser Leu Asn Ile Gln Ala Ser Gln Pro
225                 230                 235                 240

Asn Ile Pro Gln Thr Asp Glu Ser Thr Leu Met Thr Ile Ala Tyr Val
                245                 250                 255

Met Ala Ala Ile Cys Ala Leu Phe Met Leu Pro Leu Cys Leu Met Val
            260                 265                 270

Cys Gln Trp Arg Cys Leu Arg Cys Leu Arg Gln Gln His Asp Asp Phe
        275                 280                 285

Ala Asp Asp Ile Ser Leu Leu Lys Glu Gln Lys Leu Ile Ser Glu Glu
    290                 295                 300

Asp Leu
305

<210>    40
<211>    303
<212>    PRT
<213>    Artificial Sequence

<220>
<223>    SAS3

<400>    40
Met Glu Thr Asp Thr Leu Leu Leu Trp Val Leu Leu Leu Trp Val Pro
  1               5                  10                  15

Gly Ser Thr Gly Asp Tyr Pro Tyr Asp Val Pro Asp Tyr Ala Arg Asp
             20                  25                  30

Tyr Asn Pro Ile Ser Asn Asn Ile Cys His Leu Thr Asn Val Ser Asp
         35                  40                  45

Gly Ala Ser Asn Ser Leu Tyr Gly Val Gly Phe Gly Pro Leu Ile Leu
    50                  55                  60

Thr Asn Arg His Leu Phe Glu Arg Asn Asn Gly Glu Leu Val Ile Lys
 65                  70                  75                  80

Ser Arg His Gly Glu Phe Val Ile Lys Asn Thr Thr Gln Leu His Leu
                 85                  90                  95

Leu Pro Ile Pro Asp Arg Asp Leu Leu Leu Ile Arg Leu Pro Lys Asp
            100                 105                 110

Ile Pro Pro Phe Pro Gln Lys Leu Gly Phe Arg Gln Pro Glu Lys Gly
        115                 120                 125

Glu Arg Ile Cys Met Val Gly Ser Asn Phe Gln Thr Lys Ser Ile Thr
    130                 135                 140

Ser Val Val Ser Glu Thr Ser Thr Ile Met Pro Val Glu Asn Ser Gln
145                 150                 155                 160

Phe Trp Lys His Trp Ile Ser Thr Lys Asp Gly Gln Cys Gly Ser Pro
                165                 170                 175

Met Val Ser Thr Lys Asp Gly Lys Ile Leu Gly Leu His Ser Leu Ala
            180                 185                 190

Asn Phe Gln Asn Ser Ile Asn Tyr Phe Ala Ala Phe Pro Asp Asp Phe
        195                 200                 205

Ala Glu Lys Tyr Leu His Thr Ile Glu Ala His Glu Trp Val Lys His
    210                 215                 220

Trp Lys Tyr Asn Thr Ser Ala Ile Ser Trp Gly Ser Leu Asn Ile Gln
225                 230                 235                 240

Ala Ser Gln Pro Ala Val Gly Gln Asp Thr Gln Glu Val Ile Val Val
                245                 250                 255

Pro His Ser Leu Pro Phe Lys Val Val Val Ile Ser Ala Ile Leu Ala
            260                 265                 270

Leu Val Val Leu Thr Ile Ile Ser Leu Ile Ile Leu Ile Met Leu Trp
        275                 280                 285

Gln Lys Lys Pro Arg Glu Gln Lys Leu Ile Ser Glu Glu Asp Leu
    290                 295                 300

<210>    41
<211>    322
<212>    PRT
<213>    Artificial Sequence

<220>
<223>    SAS4

<400>    41
Met Thr Ser Arg Leu Ser Asp Pro Asn Ser Ser Ala Arg Ser Asp Met
 1               5                  10                  15

Ser Val Pro Leu Tyr Pro Thr Ala Ser Pro Val Ser Val Glu Ala Tyr
            20                  25                  30

Tyr Ser Glu Ser Glu Asp Glu Ala Ala Asn Asp Phe Leu Val Arg Met
        35                  40                  45

Gly Arg Gln Gln Ser Val Leu Arg Arg Arg Arg Arg Arg Thr Arg Cys
    50                  55                  60

Val Gly Met Val Ile Ala Cys Leu Leu Val Ala Val Leu Ser Gly Gly
65                  70                  75                  80

Phe Gly Ala Leu Leu Met Trp Leu Leu Arg Ser Asn Ser Met Phe Arg
                85                  90                  95

Gly Leu Arg Asp Tyr Asn Pro Ile Ser Asn Asn Ile Cys His Leu Thr
            100                 105                 110

Asn Val Ser Asp Gly Ala Ser Asn Ser Leu Tyr Gly Val Gly Phe Gly
        115                 120                 125

Pro Leu Ile Leu Thr Asn Arg His Leu Phe Glu Arg Asn Asn Gly Glu
    130                 135                 140

Leu Val Ile Lys Ser Arg His Gly Glu Phe Val Ile Lys Asn Thr Thr
145                 150                 155                 160

Gln Leu His Leu Leu Pro Ile Pro Asp Arg Asp Leu Leu Leu Ile Arg
                165                 170                 175

Leu Pro Lys Asp Ile Pro Pro Phe Pro Gln Lys Leu Gly Phe Arg Gln
            180                 185                 190

Pro Glu Lys Gly Glu Arg Ile Cys Met Val Gly Ser Asn Phe Gln Thr
        195                 200                 205

Lys Ser Ile Thr Ser Val Val Ser Glu Thr Ser Thr Ile Met Pro Val
    210                 215                 220

Glu Asn Ser Gln Phe Trp Lys His Trp Ile Ser Thr Lys Asp Gly Gln
225                 230                 235                 240

Cys Gly Ser Pro Met Val Ser Thr Lys Asp Gly Lys Ile Leu Gly Leu
                245                 250                 255

His Ser Leu Ala Asn Phe Gln Asn Ser Ile Asn Tyr Phe Ala Ala Phe
            260                 265                 270

Pro Asp Asp Phe Ala Glu Lys Tyr Leu His Thr Ile Glu Ala His Glu
        275                 280                 285

Trp Val Lys His Trp Lys Tyr Asn Thr Ser Ala Ile Ser Trp Gly Ser
    290                 295                 300

Leu Asn Ile Gln Ala Ser Gln Pro Glu Gln Lys Leu Ile Ser Glu Glu
305                 310                 315                 320

Asp Leu

<210>    42
<211>    1008
<212>    DNA
<213>    Artificial Sequence

<220>
<223>    SAS (cDNA)

<400>    42
atggctcagg ctctcccctg gcttttgctg tggatgggcg cgggagtttt gcctgctcat      60

ggtactcagc acggtattcg ccttcccctt cggagtgggt tgggcggcgc acctctggga     120

ctgcgcttgc ctagggagac agacgaggag cctgaagaac cgggtcgcga ttataatccg     180

attagtaata atatatgtca tctcacgaac gtatccgacg gtgcaagcaa cagcctgtac     240

ggagtcgggt ttgggccgct tattttgaca aaccgacatc tctttgagag aaataatggg     300

gagcttgtca tcaaaagtcg gcacggagaa tttgtaataa agaataccac acaactccat     360

cttctcccga ttccggatcg cgatcttctc ctgatcaggt tgccgaagga tatccctcca     420

ttcccgcaga aacttggatt caggcaaccg gaaaagggag aacgcatctg catggtcggg     480

agcaatttcc aaactaagtc tatcacaagc gtggtgtctg aaactagtac gataatgcca     540

gtagagaata gccagttttg gaaacattgg atcagcacga aagacggaca atgcggatct     600

ccgatggtgt caactaaaga tggtaaaata ctcggcctgc attcacttgc caacttccaa     660

aactcaatta actatttcgc cgcattccct gacgactttg ccgaaaagta ccttcacact     720

atcgaggcac atgaatgggt aaagcactgg aagtataaca ctagcgcgat ctcatggggg     780

tcactgaata ttcaagcgtc tcagccaaat atacctcaaa cggacgagag cactcttatg     840

accatagctt atgtgatggc tgcgatctgt gcgctcttca tgcttccctt gtgcctcatg 900

gtctgtcaat ggcgatgtct gaggtgcctt cgacaacaac atgatgattt tgcggacgat 960

atttctcttt tgaaagagca gaaacttatt tcagaagagg acctctga 1008

<210> 43
<211> 244
<212> PRT
<213> Artificial Sequence

<220>
<223> PVY NIa protease

<400> 43
Ala Lys Ser Leu Met Arg Gly Leu Arg Asp Phe Asn Pro Ile Ala Gln
1               5                   10                  15

Thr Val Cys Arg Leu Lys Val Ser Val Glu Tyr Gly Thr Ser Glu Met
            20                  25                  30

Tyr Gly Phe Gly Phe Gly Ala Tyr Ile Ile Ala Asn His His Leu Phe
        35                  40                  45

Arg Ser Tyr Asn Gly Ser Met Glu Val Arg Ser Met His Gly Thr Phe
    50                  55                  60

Arg Val Lys Asn Leu His Ser Leu Ser Val Leu Pro Ile Lys Gly Arg
65                  70                  75                  80

Asp Ile Ile Leu Ile Lys Met Pro Lys Asp Phe Pro Val Phe Pro Gln
                85                  90                  95

Lys Leu His Phe Arg Ala Pro Thr Gln Asn Glu Arg Val Cys Leu Val
            100                 105                 110

Gly Thr Asn Phe Gln Glu Lys Tyr Ala Ser Ser Ile Ile Thr Glu Thr
        115                 120                 125

Ser Thr Thr Tyr Asn Ile Pro Gly Ser Thr Phe Trp Lys His Trp Ile
    130                 135                 140

Glu Thr Asp Asn Gly His Cys Gly Leu Pro Val Val Ser Thr Thr Asp
145                 150                 155                 160

Gly Cys Leu Val Gly Ile His Ser Leu Ala Asn Asn Lys His Thr Thr
                165                 170                 175

Asn Tyr Tyr Ser Ala Phe Asp Glu Asp Phe Glu Ser Lys Tyr Leu Arg
            180                 185                 190

Thr Asn Glu His Asn Glu Trp Val Lys Ser Trp Ile Tyr Asn Pro Asp
        195                 200                 205

Thr Val Leu Trp Gly Pro Leu Lys Leu Lys Asp Ser Thr Pro Lys Gly
    210                 215                 220

Leu Phe Lys Thr Thr Lys Leu Val Gln Asp Leu Ile Asp His Asp Val
225                 230                 235                 240

Val Val Glu Gln

<210> 44
<211> 210
<212> PRT
<213> Artificial Sequence

<220>
<223> PVY NIa ND8CD26

<400> 44
Arg Asp Phe Asn Pro Val Ala Gln Thr Val Cys Arg Leu Lys Val Ser
1               5                   10                  15

Val Glu Phe Gly Thr Ser Glu Leu Tyr Gly Ile Gly Phe Gly Ala Tyr
            20                  25                  30

Ile Ile Ala Asn His His Leu Phe Lys Ser Tyr Asn Gly Ser Met Glu
        35                  40                  45

Val Arg Ser Met His Gly Thr Phe Arg Val Lys Asn Leu His Ser Leu
    50                  55                  60

Ser Val Leu Pro Ile Lys Gly Arg Asp Ile Ile Leu Ile Lys Met Pro
65                  70                  75                  80

Lys Asp Phe Pro Val Phe Pro Gln Lys Leu Arg Phe Arg Ala Pro Lys
                85                  90                  95

Gln Asn Glu Arg Ile Cys Leu Val Gly Thr Asn Phe Gln Glu Lys Tyr
            100                 105                 110

Ala Ser Ser Ile Ile Thr Glu Thr Ser Thr Thr Tyr Asn Val Pro Gly
        115                 120                 125

Ser Thr Phe Trp Lys His Trp Ile Glu Thr Asp Asp Gly His Cys Gly
    130                 135                 140

Leu Pro Val Val Ser Thr Ala Asp Gly Cys Leu Val Gly Ile His Ser
145                 150                 155                 160

Leu Ala Asn Asn Val Gln Thr Thr Asn Tyr Tyr Ser Ala Phe Asp Glu
                165                 170                 175

Asp Phe Glu Ser Lys Tyr Leu Arg Asn Asn Glu His Asn Glu Trp Ile
            180                 185                 190

Lys Ser Trp Val Tyr Asn Pro Asp Thr Val Leu Trp Gly Pro Leu Lys
        195                 200                 205

Leu Lys
    210

<210> 45
<211> 244
<212> PRT
<213> Artificial Sequence

<220>

<223>    ScMV NIa protease

<400>    45
Ser Lys Ser Leu Met Lys Gly Leu Arg Asp Tyr Asn Pro Ile Ala Gln
1               5                   10                  15

Thr Ile Cys Lys Leu Lys Val Thr Ser Glu Phe Gly Thr Ser Glu Met
            20                  25                  30

Tyr Gly Ile Gly Phe Gly Ala Tyr Ile Ile Ser Asn His His Leu Phe
        35                  40                  45

Lys Ser Phe Asn Gly Thr Leu Glu Val Arg Ser His His Gly Thr Phe
    50                  55                  60

Lys Val Asn Asn Met Met Ala Leu Gln Val Lys Pro Ile Ala Gly Arg
65                  70                  75                  80

Asp Ile Ile Ile Ile Lys Met Pro Lys Asp Phe Pro Val Phe Pro Gln
                85                  90                  95

Lys Leu His Phe Arg Ser Pro Lys Asn Asn Glu Arg Met Cys Ile Val
            100                 105                 110

Gly Thr Asn Phe Gln Glu Lys Ser Ala Ser Ser Thr Ile Thr Glu Thr
        115                 120                 125

Ser Ala Thr Tyr Met Val Pro Arg Ser Ser Phe Trp Lys His Trp Ile
    130                 135                 140

Ala Thr Asp Asp Gly His Cys Gly Leu Pro Val Val Ser Thr Ile Asp
145                 150                 155                 160

Gly Lys Ile Ile Gly Ile His Ser Leu Ala Asn Asn Ala Asn Ser Glu
                165                 170                 175

Asn Tyr Tyr Ala Ala Phe Asp Asp Asp Phe Glu Glu Lys Phe Leu Arg
            180                 185                 190

Thr Ser Glu His Thr Asp Trp Val Lys Asn Trp Arg Tyr Asn Pro Asp
        195                 200                 205

Thr Val Val Trp Gly Pro Leu Lys Leu Thr Glu Ser Thr Pro Lys Gly
    210                 215                 220

Leu Phe Lys Pro Thr Lys Ile Leu Glu Asp Leu Phe Glu Tyr Asn Ser
225                 230                 235                 240

Val Arg Glu Gln

<210>    46
<211>    210
<212>    PRT
<213>    Artificial Sequence

<220>
<223>    ScMV NIa ND8CD26

<400>    46

Arg Asp Tyr Asn Pro Ile Ala Gln Thr Ile Cys Lys Leu Lys Val Thr
1               5                   10                  15

Ser Glu Phe Gly Thr Ser Glu Met Tyr Gly Ile Gly Phe Gly Ala Tyr
            20                  25                  30

Ile Ile Ser Asn His His Leu Phe Lys Ser Phe Asn Gly Thr Leu Glu
        35                  40                  45

Val Arg Ser His His Gly Thr Phe Lys Val Asn Asn Met Met Ala Leu
    50                  55                  60

Gln Val Lys Pro Ile Ala Gly Arg Asp Ile Ile Ile Ile Lys Met Pro
65                  70                  75                  80

Lys Asp Phe Pro Val Phe Pro Gln Lys Leu His Phe Arg Ser Pro Lys
                85                  90                  95

Asn Asn Glu Arg Met Cys Ile Val Gly Thr Asn Phe Gln Glu Lys Ser
            100                 105                 110

Ala Ser Ser Thr Ile Thr Glu Thr Ser Ala Thr Tyr Met Val Pro Arg
        115                 120                 125

Ser Ser Phe Trp Lys His Trp Ile Ala Thr Asp Asp Gly His Cys Gly
    130                 135                 140

Leu Pro Val Val Ser Thr Ile Asp Gly Lys Ile Ile Gly Ile His Ser
145                 150                 155                 160

Leu Ala Asn Asn Ala Asn Ser Glu Asn Tyr Tyr Ala Ala Phe Asp Asp
                165                 170                 175

Asp Phe Glu Glu Lys Phe Leu Arg Thr Ser Glu His Thr Asp Trp Val
            180                 185                 190

Lys Asn Trp Arg Tyr Asn Pro Asp Thr Val Val Trp Gly Pro Leu Lys
        195                 200                 205

Leu Thr
    210


<210>   47
<211>   246
<212>   PRT
<213>   Artificial Sequence

<220>
<223>   WpMV NIa protease


<400>   47
Ala Lys Ser Leu Leu Lys Gly Leu Arg Asp Tyr Asn Ala Thr Ala Gln
1               5                   10                  15

Thr Val Cys Leu Leu Thr Val Glu Thr Glu Val Gly Gln Thr Thr Thr
            20                  25                  30

His Gly Ile Gly Phe Gly Ser Leu Leu Val Ala Asn His His Leu Phe
        35                  40                  45

Lys Ser Phe Asn Gly Thr Leu Thr Val Arg Ser His His Gly Thr Phe

50                  55                  60

Lys Val Pro Asn Leu Met Gln Leu Gln Val Gln Pro Leu Lys Gly Arg
65                  70                  75                  80

Asp Leu Ile Val Ile Lys Met Pro Lys Asp Phe Pro Val Phe Pro Gln
                85                  90                  95

Arg Leu His Phe Arg Ala Pro Thr Gln Gln Asp Arg Val Val Leu Val
            100                 105                 110

Gly Ser Asn Phe Gln Glu Lys Tyr Ile Ser Thr Thr Leu Ser Glu Thr
        115                 120                 125

Ser Ser Thr Phe Pro Val Gln Arg Ser Ser Phe Trp Lys His Trp Ile
    130                 135                 140

Ser Thr Asp Asp Gly Asn Cys Gly Leu Pro Leu Val Ser Thr Ala Asp
145                 150                 155                 160

Gly Tyr Ile Leu Gly Leu His Ser Leu Ala Asn Asn Arg Asn Ser Glu
                165                 170                 175

Asn Tyr Phe Ala Ala Phe Asp Asp Glu Phe Glu Asp Arg Tyr Leu Arg
            180                 185                 190

Thr Glu Glu His Ala Gln Trp Thr Arg Asn Trp Lys Tyr Asn Pro Asp
        195                 200                 205

Asn Val Leu Trp Gly Ser Leu Lys Leu Thr Lys Ser Thr Pro Asp Gly
    210                 215                 220

Met Phe Lys Thr Thr Lys Met Ile Glu Asp Leu Ala Ala Phe Lys Ile
225                 230                 235                 240

Asp Glu Val His Glu Gln
                245


<210>   48
<211>   210
<212>   PRT
<213>   Artificial Sequence

<220>
<223>   WpMV NIa ND8CD28


<400>   48
Arg Asp Tyr Asn Ala Thr Ala Gln Thr Val Cys Leu Leu Thr Val Glu
1               5                   10                  15

Thr Glu Val Gly Gln Thr Thr Thr His Gly Ile Gly Phe Gly Ser Leu
            20                  25                  30

Leu Val Ala Asn His His Leu Phe Lys Ser Phe Asn Gly Thr Leu Thr
        35                  40                  45

Val Arg Ser His His Gly Thr Phe Lys Val Pro Asn Leu Met Gln Leu
    50                  55                  60

Gln Val Gln Pro Leu Lys Gly Arg Asp Leu Ile Val Ile Lys Met Pro
65                  70                  75                  80

Lys Asp Phe Pro Val Phe Pro Gln Arg Leu His Phe Arg Ala Pro Thr
                85                  90                  95

Gln Gln Asp Arg Val Val Leu Val Gly Ser Asn Phe Gln Glu Lys Tyr
            100                 105                 110

Ile Ser Thr Thr Leu Ser Glu Thr Ser Ser Thr Phe Pro Val Gln Arg
        115                 120                 125

Ser Ser Phe Trp Lys His Trp Ile Ser Thr Asp Asp Gly Asn Cys Gly
    130                 135                 140

Leu Pro Leu Val Ser Thr Ala Asp Gly Tyr Ile Leu Gly Leu His Ser
145                 150                 155                 160

Leu Ala Asn Asn Arg Asn Ser Glu Asn Tyr Phe Ala Ala Phe Asp Asp
                165                 170                 175

Glu Phe Glu Asp Arg Tyr Leu Arg Thr Glu Glu His Ala Gln Trp Thr
            180                 185                 190

Arg Asn Trp Lys Tyr Asn Pro Asp Asn Val Leu Trp Gly Ser Leu Lys
        195                 200                 205

Leu Thr
    210


<210>    49
<211>    336
<212>    PRT
<213>    Artificial Sequence

<220>
<223>    SAS(PVY)


<400>    49
Met Ala Gln Ala Leu Pro Trp Leu Leu Leu Trp Met Gly Ala Gly Val
 1               5                  10                  15

Leu Pro Ala His Gly Thr Gln His Gly Ile Arg Leu Pro Leu Arg Ser
            20                  25                  30

Gly Leu Gly Gly Ala Pro Leu Gly Leu Arg Leu Pro Arg Glu Thr Asp
        35                  40                  45

Glu Glu Pro Glu Glu Pro Gly Leu Gln Arg Asp Phe Asn Pro Val Ala
    50                  55                  60

Gln Thr Val Cys Arg Leu Lys Val Ser Val Glu Phe Gly Thr Ser Glu
65                  70                  75                  80

Leu Tyr Gly Ile Gly Phe Gly Ala Tyr Ile Ile Ala Asn His His Leu
                85                  90                  95

Phe Lys Ser Tyr Asn Gly Ser Met Glu Val Arg Ser Met His Gly Thr
            100                 105                 110

Phe Arg Val Lys Asn Leu His Ser Leu Ser Val Leu Pro Ile Lys Gly
        115                 120                 125

Arg Asp Ile Ile Leu Ile Lys Met Pro Lys Asp Phe Pro Val Phe Pro
    130                 135                 140

Gln Lys Leu Arg Phe Arg Ala Pro Lys Gln Asn Glu Arg Ile Cys Leu
145                 150                 155                 160

Val Gly Thr Asn Phe Gln Glu Lys Tyr Ala Ser Ser Ile Ile Thr Glu
                165                 170                 175

Thr Ser Thr Thr Tyr Asn Val Pro Gly Ser Thr Phe Trp Lys His Trp
            180                 185                 190

Ile Glu Thr Asp Asp Gly His Cys Gly Leu Pro Val Val Ser Thr Ala
        195                 200                 205

Asp Gly Cys Leu Val Gly Ile His Ser Leu Ala Asn Asn Val Gln Thr
    210                 215                 220

Thr Asn Tyr Tyr Ser Ala Phe Asp Glu Asp Phe Glu Ser Lys Tyr Leu
225                 230                 235                 240

Arg Asn Asn Glu His Asn Glu Trp Ile Lys Ser Trp Val Tyr Asn Pro
                245                 250                 255

Asp Thr Val Leu Trp Gly Pro Leu Lys Leu Lys Ala Ala Ala Asn Ile
            260                 265                 270

Pro Gln Thr Asp Glu Ser Thr Leu Met Thr Ile Ala Tyr Val Met Ala
        275                 280                 285

Ala Ile Cys Ala Leu Phe Met Leu Pro Leu Cys Leu Met Val Cys Gln
    290                 295                 300

Trp Arg Cys Leu Arg Cys Leu Arg Gln Gln His Asp Asp Phe Ala Asp
305                 310                 315                 320

Asp Ile Ser Leu Leu Lys Glu Gln Lys Leu Ile Ser Glu Glu Asp Leu
                325                 330                 335

<210>    50
<211>    336
<212>    PRT
<213>    Artificial Sequence

<220>
<223>    SAS(ScMV)

<400>    50
Met Ala Gln Ala Leu Pro Trp Leu Leu Leu Trp Met Gly Ala Gly Val
 1               5                  10                  15

Leu Pro Ala His Gly Thr Gln His Gly Ile Arg Leu Pro Leu Arg Ser
            20                  25                  30

Gly Leu Gly Gly Ala Pro Leu Gly Leu Arg Leu Pro Arg Glu Thr Asp
        35                  40                  45

Glu Glu Pro Glu Glu Pro Gly Leu Gln Arg Asp Tyr Asn Pro Ile Ala
    50                  55                  60

Gln Thr Ile Cys Lys Leu Lys Val Thr Ser Glu Phe Gly Thr Ser Glu
65                  70                  75                  80

Met Tyr Gly Ile Gly Phe Gly Ala Tyr Ile Ile Ser Asn His His Leu
                85                  90                  95

Phe Lys Ser Phe Asn Gly Thr Leu Glu Val Arg Ser His His Gly Thr
            100                 105                 110

Phe Lys Val Asn Asn Met Met Ala Leu Gln Val Lys Pro Ile Ala Gly
        115                 120                 125

Arg Asp Ile Ile Ile Ile Lys Met Pro Lys Asp Phe Pro Val Phe Pro
    130                 135                 140

Gln Lys Leu His Phe Arg Ser Pro Lys Asn Asn Glu Arg Met Cys Ile
145                 150                 155                 160

Val Gly Thr Asn Phe Gln Glu Lys Ser Ala Ser Ser Thr Ile Thr Glu
                165                 170                 175

Thr Ser Ala Thr Tyr Met Val Pro Arg Ser Ser Phe Trp Lys His Trp
            180                 185                 190

Ile Ala Thr Asp Asp Gly His Cys Gly Leu Pro Val Val Ser Thr Ile
        195                 200                 205

Asp Gly Lys Ile Ile Gly Ile His Ser Leu Ala Asn Asn Ala Asn Ser
    210                 215                 220

Glu Asn Tyr Tyr Ala Ala Phe Asp Asp Asp Phe Glu Glu Lys Phe Leu
225                 230                 235                 240

Arg Thr Ser Glu His Thr Asp Trp Val Lys Asn Trp Arg Tyr Asn Pro
                245                 250                 255

Asp Thr Val Val Trp Gly Pro Leu Lys Leu Thr Ala Ala Ala Asn Ile
            260                 265                 270

Pro Gln Thr Asp Glu Ser Thr Leu Met Thr Ile Ala Tyr Val Met Ala
        275                 280                 285

Ala Ile Cys Ala Leu Phe Met Leu Pro Leu Cys Leu Met Val Cys Gln
    290                 295                 300

Trp Arg Cys Leu Arg Cys Leu Arg Gln Gln His Asp Asp Phe Ala Asp
305                 310                 315                 320

Asp Ile Ser Leu Leu Lys Glu Gln Lys Leu Ile Ser Glu Glu Asp Leu
                325                 330                 335

<210>    51
<211>    336
<212>    PRT
<213>    Artificial Sequence

<220>
<223>    SAS(WpMV)

<400>    51

Met Ala Gln Ala Leu Pro Trp Leu Leu Leu Trp Met Gly Ala Gly Val
1               5                   10                  15

Leu Pro Ala His Gly Thr Gln His Gly Ile Arg Leu Pro Leu Arg Ser
            20                  25                  30

Gly Leu Gly Gly Ala Pro Leu Gly Leu Arg Leu Pro Arg Glu Thr Asp
        35                  40                  45

Glu Glu Pro Glu Glu Pro Gly Leu Gln Arg Asp Tyr Asn Ala Thr Ala
    50                  55                  60

Gln Thr Val Cys Leu Leu Thr Val Glu Thr Glu Val Gly Gln Thr Thr
65                  70                  75                  80

Thr His Gly Ile Gly Phe Gly Ser Leu Leu Val Ala Asn His His Leu
                85                  90                  95

Phe Lys Ser Phe Asn Gly Thr Leu Thr Val Arg Ser His His Gly Thr
            100                 105                 110

Phe Lys Val Pro Asn Leu Met Gln Leu Gln Val Gln Pro Leu Lys Gly
        115                 120                 125

Arg Asp Leu Ile Val Ile Lys Met Pro Lys Asp Phe Pro Val Phe Pro
    130                 135                 140

Gln Arg Leu His Phe Arg Ala Pro Thr Gln Gln Asp Arg Val Val Leu
145                 150                 155                 160

Val Gly Ser Asn Phe Gln Glu Lys Tyr Ile Ser Thr Thr Leu Ser Glu
                165                 170                 175

Thr Ser Ser Thr Phe Pro Val Gln Arg Ser Ser Phe Trp Lys His Trp
            180                 185                 190

Ile Ser Thr Asp Asp Gly Asn Cys Gly Leu Pro Leu Val Ser Thr Ala
        195                 200                 205

Asp Gly Tyr Ile Leu Gly Leu His Ser Leu Ala Asn Asn Arg Asn Ser
    210                 215                 220

Glu Asn Tyr Phe Ala Ala Phe Asp Asp Glu Phe Glu Asp Arg Tyr Leu
225                 230                 235                 240

Arg Thr Glu Glu His Ala Gln Trp Thr Arg Asn Trp Lys Tyr Asn Pro
                245                 250                 255

Asp Asn Val Leu Trp Gly Ser Leu Lys Leu Thr Ala Ala Ala Asn Ile
            260                 265                 270

Pro Gln Thr Asp Glu Ser Thr Leu Met Thr Ile Ala Tyr Val Met Ala
        275                 280                 285

Ala Ile Cys Ala Leu Phe Met Leu Pro Leu Cys Leu Met Val Cys Gln
    290                 295                 300

Trp Arg Cys Leu Arg Cys Leu Arg Gln Gln His Asp Asp Phe Ala Asp
305                 310                 315                 320

Asp Ile Ser Leu Leu Lys Glu Gln Lys Leu Ile Ser Glu Glu Asp Leu
                325                 330                 335

## Description

### Technical Field

The present invention relates to synthetic α-secretase and a use thereof.

### Background Art

Dementia means that the gradual loss of rational thinking and the ability to engage in social activities becomes serious enough to cause an obstacle in a person's daily life. Dementia can be largely divided into dementia caused by Alzheimer's disease, Parkinson's disease, Huntington's disease, and vascular dementia caused by blockage or narrowing of blood vessels, and among these, Alzheimer's disease is the most common cause of dementia.

Drugs used to treat Alzheimer's disease can largely be classified into antioxidants, anti-inflammatory drugs, female hormones, and acetylcholinesterase inhibitors. However, most of these drugs have serious side effects, and also have limitations in that they are drugs for relieving symptoms instead of treating the underlying cause of Alzheimer's disease. Therefore, there is an urgent need to develop therapeutic agents for the fundamental treatment of Alzheimer's disease.

Alzheimer's disease is anatomically characterized by amyloid plaques generated outside of brain cells and neurofibrillary tangles (NFTs) generated within the cells. Amyloid plaques consist of a small peptide called amyloid β (Aβ), which is known to be a major cause of Alzheimer's disease.

Specifically, amyloid β, which is a major component forming amyloid plaques in the brain of Alzheimer's disease patients, is a peptide composed of 39 to 43 amino acids and is also observed in patients with Lewy body dementia, inclusion body myositis, or myopathy. Additionally, amyloid β forms aggregates coating cerebral blood vessels in patients with cerebral amyloid angiopathy. These amyloid plaques are protein folds shared by other peptides (*e.g*., prions) and consist of bundles of well-ordered fibrillar aggregates called amyloid fibers. The water-soluble site of amyloid β peptide, which is formed by cleavage from amyloid precursor protein (APP), acts act as a determining factor in the progression of Alzheimer's disease. The formation of this amyloid β is achieved through the degradation of amyloid precursor protein by β-secretase and γ-secretase. On the other hand, when amyloid precursor protein is degraded by α-secretase (which competes with β-secretase) instead of β-secretase, the formation of amyloid β is inhibited (LaFerla FM *et al.,* 2007).

The α-secretase cleaves the amyloid precursor protein to produce soluble APPα (hereinafter "sAPPα"). The produced sAPPα has a neuroprotective effect and plays an important role in the survival of neurons (Christian Tackenberg et al., Molecular Brainvolume 12, 27 (2019)). In contrast, the β-secretase cleaves the amyloid precursor protein to produce soluble APPβ (hereinafter "sAPPβ") and amyloid β. The produced sAPPβ and amyloid β are known to cause neurotoxicity. Therefore, development of β-secretase and γ-secretase inhibitors and substances capable of activating α-secretase are in progress as a method to inhibit the formation of amyloid β. Studies on amyloid β-degrading enzymes to remove already formed amyloid β in different directions are underway.

### Disclosure of Invention

### Technical Problem

The present invention is to provide a novel fusion protein which includes NIa protease or a fragment thereof, or a variant thereof, and a therapeutic agent for amyloid β-associated diseases prepared using the same.

### Solution to Problem

In order to achieve the object, the present invention provides a fusion protein of Formula 1 below:

[Formula 1] (X)ₘ - (L1)ₙ - A - (L2)ₒ - (Y)p - (Z)q

in which in Formula 1 above,
X is a signal sequence;
L1 is peptide linker 1;
A is a nuclear inclusion a (NIa) protease or a fragment thereof, or a variant thereof;
L2 is peptide linker 2;
Y is a transmembrane domain;
Z is an intracellular domain; and
m, n, o, p, and q are each 0 or 1.

Additionally, the present invention provides a polynucleotide encoding the fusion protein.

Additionally, the present invention provides an expression vector including the polynucleotide.

Additionally, the present invention provides a recombinant virus including the polynucleotide.

Additionally, the present invention provides a host cell transfected with an expression vector.

Additionally, the present invention provides a pharmaceutical composition for preventing or treating diseases caused by amyloid β, which includes the expression vector as an active ingredient.

Additionally, the present invention provides a method for preventing or treating diseases caused by amyloid β, which includes administering the composition to an individual.

### Advantageous Effects of Invention

The synthetic α-secretase (hereinafter, SAS), which is a fusion protein according to the present invention that includes NIa protease, a fragment thereof, or a variant thereof as an active ingredient, can inhibit the formation of amyloid β, can degrade amyloid β secreted extracellularly, and can also degrade amyloid β that is introduced into cells from the outside. Therefore, the present invention can be utilized for degrading amyloid β, which is the cause of various diseases including Alzheimer's disease, thereby preventing or treating these diseases.

### Brief Description of Drawings

FIG. 1 shows a drawing confirming the activity of a fragment of NIa using amyloid β as a substrate.
FIG. 2 shows a schematic drawing illustrating the structure of SAS. NIa protease is a protein present in the cytoplasm. However, SAS, which is a novel fusion protein including NIa protease, a fragment thereof, or a variant thereof, is expressed and acts in the protein secretion pathway including the endoplasmic reticulum. In particular, ss stands for a signal sequence.
FIG. 3 shows a schematic drawing illustrating the structure of a fusion protein sub(+), which includes a substrate sequence in which SAS can act, and the structure of a fusion protein sub(-), where PI (histidine) and P2 (glutamine) are designed to prevent SAS from acting by substituting these parts with alanine.
FIG. 4 shows a drawing confirming that SAS has an α-secretase activity that specifically cleaves the amyloid β sequence using the fusion protein sub(+) and sub(-) shown in FIG. 3 as substrates through the Western blot technique.
FIG. 5 shows a schematic drawing illustrating the structure of an amyloid precursor protein from which amyloid β is derived, in which the sites cleaved by α-secretase, β-secretase, and SAS are indicated.
FIG. 6 shows a schematic drawing illustrating the structures of SAS and a few variants of SAS.
FIG. 7 shows a drawing illustrating the measurement results of activities of SAS or SAS variants through the Western blot technique using the sAPPα antibody, after transfecting HEK293T cells with a plasmid encoding an amyloid precursor protein and SAS or a variant of SAS.
FIG. 8 shows a drawing confirming the N-glycosylation of SAS within HEK293T cells using EndoH and PngF (*i.e.,* enzymes for removing N-glycosylation) through the Western blot technique.

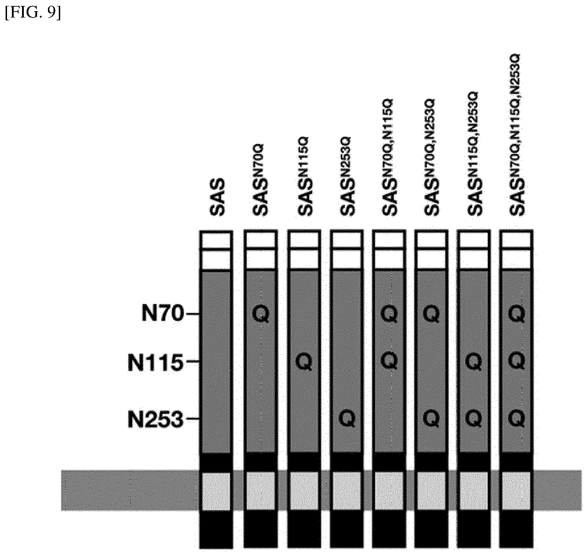

FIG. 9 shows a schematic drawing illustrating the positions for asparagine (N70, N115, or N253) which are the sites where the N-glycosylation occurs, and variants where N70, N115, or N253 was substituted with glutamine (Q) to prevent the occurrence of N-glycosylation.
FIG. 10 shows a drawing illustrating the measurement results of activities of SAS and various SAS variants, in which N-glycosylation was inhibited, through the Western blot technique using the sAPPα antibody.
FIG. 11 shows a drawing confirming that not only fusion proteins prepared using the NIa protease derived from TuMV, but also fusion proteins prepared using the NIa protease derived from *Potato Virus Y* (PVY), *Sunflower Chlorotic Mottle Virus* (ScMV), and *Wild Potato Mosaic Virus* (WpMV) can have the activity as an SAS, through the Western blot technique using the sAPPα antibody.
FIG. 12a shows a graph illustrating the open field test (OFT) results with regard to the 5XFAD mice of the NL-EGFP group, NL-SAS group, TG-EGFP group, and TG-SAS group.
FIG. 12b shows a graph illustrating the novel object recognition (NOR) test results with regard to the 5XFAD mice of the NL-EGFP group, NL-SAS group, TG-EGFP group, and TG-SAS group (^{∗} : p < 0.05, ^{∗∗} : p < 0.01).
FIG. 12c shows a graph illustrating the Morris water maze (MWM) test results with regard to the 5XFAD mice of the NL-EGFP group, NL-SAS group, TG-EGFP group, and TG-SAS group, in which the time taken to find the location of the platform for each date (^{∗} : p < 0.05, ^{∗∗} : p < 0.01, ^{∗∗∗} : p < 0.005).
FIG. 12d shows a drawing illustrating the paths taken by the mice in each group in the final test on the second day of reversal of the Morris water maze (MWM) test with regard to the 5XFAD mice of the NL-EGFP group, NL-SAS group, TG-EGFP group, and TG-SAS group.
FIG. 12e shows a graph illustrating the measurement results regarding the amount of time each group of mice stayed in the original quadrant of the platform on the second day of reversal of the Morris water maze (MWM) test with regard to the 5XFAD mice of the NL-EGFP group, NL-SAS group, TG-EGFP group, and TG-SAS group (^{∗} : p < 0.05, ^{∗∗∗} : p < 0.005).
FIG. 13a shows a photographic image taken using a fluorescence microscope after staining the plaques in the brain tissue of a 5XFAD mouse in the TG-EGFP group using the Aβ antibody.
FIG. 13b shows a photographic image taken using a fluorescence microscope after staining the plaques in the brain tissue of a 5XFAD mouse in the TG-SAS group using the Aβ antibody.
FIG. 13c shows a graph illustrating the results of the number of plaques in the hippocampus region of the brain calculated by quantitative analysis after staining the plaques in the brain tissue of 5XFAD mice in the TG-EGFP group and the TG-SAS group using the Aβ antibody (^{∗∗∗} : p < 0.005).
FIG. 13d shows a graph illustrating the results of the proportion of area occupied by the plaques in the hippocampus region of the brain calculated by quantitative analysis after staining the plaques in the brain tissue of 5XFAD mice in the TG-EGFP group and the TG-SAS group using the Aβ antibody (^{∗∗∗} : p < 0.005).

### Best Mode for Carrying Out the Invention

### SAS a fusion protein

In one aspect, the present invention provides a fusion protein which includes NIa protease or a fragment thereof.

In a specific embodiment, the present invention provides a fusion protein which includes NIa protease or a fragment thereof; and a transmembrane domain.

In a specific embodiment, the fusion protein may be a fusion protein having the structure of Formula 1 below:

[Formula 1] (X)ₘ - (L1)ₙ - A - (L2)ₒ - (Y)p - (Z)q

in which in Formula 1 above,
X may be a signal sequence; L1 may be peptide linker 1; A may be a nuclear inclusion a NIa protease or a fragment thereof, or a variant thereof; L2 may be peptide linker 2; Y may be a transmembrane domain; Z may be an intracellular signaling domain; and m, n, o, p, and q may be each 0 or 1.

Specifically, the X, which is a signal sequence, may be derived from β-secretase.

The β-secretase may be a polypeptide having the amino acid sequence represented by SEQ ID NO: 1, and the gene encoding the same may be a polynucleotide having the nucleotide sequence of SEQ ID NO: 2.

Specifically, the X may be an amino acid at positions 1 to 21 in the amino acid sequence of β-secretase represented by SEQ ID NO: 1. The X may be a polypeptide having the amino acid sequence of SEQ ID NO: 3. In the fusion protein of the present invention, the X may be present or absent.

In the present invention, the L1 refers to peptide linker 1. The peptide linker 1 may be a peptide consisting of 1 to 24 amino acids. Specifically, the linker may be a peptide consisting of 1 to 24, 2 to 20, 3 to 15, or 5 to 10 amino acids, and more specifically, a peptide consisting of 3 to 24 amino acids.

In an embodiment, the linker may be a polypeptide represented by an amino acid sequence selected from the group consisting of GTDLVSIPHGPNVTVRANIAAI (SEQ ID NO: 5), DLVSIPHGPNVTVRANIAAI (SEQ ID NO: 6), DLVSIPHGPNVTVRANIA (SEQ ID NO: 7), VSIPHGPNVTVRANIA (SEQ ID NO: 8), IPHGPNVTVRANIA (SEQ ID NO: 9), IPHGPNVTVRAN (SEQ ID NO: 10), and HGPNVTVRAN (SEQ ID NO: 11), but the linker is not limited thereto. The linker merely has a role of linking these monomers, and it has a very limited effect on the structure and impact.

In a specific embodiment, the L1 may be a pro-domain of β-secretase. Specifically, the pro-domain of β-secretase may be amino acids at positions 22 to 45 (SEQ ID NO: 4) in the amino acid sequence of β-secretase represented by SEQ ID NO: 1. In the fusion protein of the present invention, the L1 may be present or absent.

The A may be NIa protease. As used herein, the term "NIa protease" has an activity of effectively degrading amyloid β. The NIa protease may be derived from the family *Potyviridae.* Preferably, the NIa protease derived from the family *Potyviridae* may be able to specifically cleave the valine-X-histidine-glutamine (VXHQ) site. In particular, the X may be any one selected from 20 amino acids. Specifically, the NIa protease may be one derived from *Agropyron mosaic virus, Algerian watermelon mosaic virus, Alstroemeria mosaic virus, Alternanthera latent virus, Amaranthus leaf mottle virus, Amazon lily mosaic virus, Angelica virus Y, Apium virus Y, Arracacha mottle virus, Basella rugose mosaic virus(=Peace lily mosaic virus), Bermuda grass southern mosaic virus, Bidens mosaic virus, Bidens mottle virus, Brugmansia suaveolens mottle virus, Brugmansia mosaic virus, Canna yellow streak virus, Carrot thin leaf virus, Carrot virus Y, Catharanthus mosaic virus, Celery mosaic virus, Celery yellow mosaic virus, Chilli ringspot virus, Chilli veinal mottle virus, Chinese artichoke mosaic virus, Christmas bell potyvirus, Cocksfoot streak virus, Commelina mild mosaic virus, Cotyledon virus Y, Cypripedium virus Y, Delphinium vein clearing virus, Donkey orchid virus A, Ecuadorian rocoto virus, Endive necrotic mosaic virus, Euphorbia ringspot virus, Gloriosa stripe mosaic virus, Henbane mosaic virus, Hordeum mosaic virus, Hyacinth mosaic virus, Ipomoea vein mosaic virus, Iris mild mosaic virus, Japanese yam mosaic virus, Johnsongrass mosaic virus, Kalanchoe mosaic virus, Keunjorong mosaic virus, Konjac mosaic virus, Lettuce mosaic virus, Lupin mosaic virus, Maize dwarf mosaic virus, Malva vein clearing virus, Moroccan watermelon mosaic virus, Muscari mosaic virus, Narcissus late season yellows virus, Narcissus yellow stripe virus, Omphalodes virus Y, Ornamental onion stripe mosaic virus, Ornithogalum mosaic virus, Ornithogalum stripe mosaic virus, Ornithogalum virus 2, Panax notoginseng virus Y, Papaya leaf distortion mosaic virus, Papaya ringspot virus, Pennisetum mosaic virus, Pepper mottle virus, Pepper severe mosaic virus, Pepper veinal mottle virus, Pepper yellow mosaic virus, Peru tomato mosaic virus, Pfaffia mosaic virus, Pleione virus Y, Plum pox virus, Potato virus V, Potato virus Y, Ranunculus mild mosaic virus, Ryegrass mosaic virus, Scallion mosaic virus, Sorghum mosaic virus, Sugarcane mosaic virus, Sunflower chlorotic mottle virus, Sweet potato feathery mottle virus, Sweet potato latent virus, Sweet potato virus 2, Sweet potato virus G, Tobacco vein banding mosaic virus, Tomato necrotic stunt virus, Trillium crinkled leaf virus, Tuberose mild mosaic virus, Tuberose mild mottle virus, Turnip mosaic virus, Unnamed Verbena potyvirus, Vallota mosaic virus, Vanilla distortion mosaic virus, Verbena virus Y, Wild potato mosaic virus, Wild tomato mosaic virus, Yam mild mosaic virus, Yam mosaic virus, Zantedeschia mosaic virus, Zea mosaic virus (= Iranian Johnsongrass mosaic virus*)*,* or *Zucchini yellow fleck virus,* but is not limited thereto.

In a specific embodiment, the NIa protease may be from Turnip mosaic virus (TuMV). TuMV is a pathogenic plant virus in plants of the family *Brassicaceae,* which is infected by 40 to 50 different kinds of aphids, and the infected plants show symptoms such as chlorotic local lesions, mosaics, stains, and wrinkles. TuMV, which has no envelope, is a positive-sense single-stranded RNA virus composed of a helical capsid with an average length of 720 nm. The genome of TuMV consists of one open reading frame (ORF) of about 10 kb and is in the form of a linear monopartite.

The TuMV-derived NIa protease may be a polypeptide consisting of 243 amino acids represented by SEQ ID NO: 12. In an embodiment, in the present invention, the TuMV-derived NIa protease may be a polypeptide (SEQ ID NO: 13) consisting of 235 amino acids, in which the 1st to the 8th amino acids are excluded from the amino acid sequence represented by SEQ ID NO: 12.

In the present invention, the TuMV-derived NIa protease may have an amino acid sequence consisting of amino acids at positions 56 to 269 in the amino acid sequence of the SAS represented by SEQ ID NO: 31. Specifically, the NIa protease may be a polypeptide having an amino acid sequence represented by SEQ ID NO: 15.

Additionally, the A may be a TuMV-derived NIa protease fragment. In an embodiment, the NIa protease fragment may be in a form in which 18 to 30 amino acid residues, and specifically 20, 21, 22, 23, 24, 25, 26, 27, or 28 amino acid residues in a direction from the C-terminus to the N-terminus in the amino acid sequence of the NIa protease represented by SEQ ID NO: 13 may be consecutively deleted. In a specific embodiment of the present invention, amyloid β-degrading activity was confirmed in the NIa protease fragments (SEQ ID NOS: 14, 16, and 17, respectively), in which 20, 24, and 28 amino acid residues were deleted from the C-terminus.

In a specific embodiment of the present invention, the TuMV-derived NIa protease fragment may be a peptide consisting of 200 to 250 amino acids in the amino acid sequence of the NIa protease represented by SEQ ID NO: 12 or 13. Specifically, the NIa protease fragment may be a polypeptide having any one of the amino acid sequences represented by SEQ ID NO: 12 to SEQ ID NO: 21.

Additionally, the A may be a TuMV-derived NIa protease variant. The NIa protease variant of the present invention may be one which, while exhibiting a biological activity equivalent to that of the NIa protease of the present invention, the amino acid sequence of wild-type NIa is mutated. In the present invention, the expression "exhibiting a biological activity equivalent to NIa protease" means that even if a specific amino acid in the amino acid sequence of NIa protease is substituted, deleted, or mutated, the NIa protease still has the activity of degrading amyloid β as in wild-type NIa protease.

Such amino acid mutations are made based on the relative similarity of amino acid side chain substituents in terms of hydrophobicity, hydrophilicity, charge, size, *etc.* By the analysis of the size, shape, and type of the amino acid side chain substituents, it can be seen that arginine, lysine, and histidine are all positively charged residues; alanine, glycine, and serine have a similar size; and phenylalanine, tryptophan, and tyrosine have a similar shape. Therefore, based on foregoing, arginine, lysine, and histidine; alanine, glycine, and serine; and phenylalanine, tryptophan, and tyrosine may be considered as biologically functional equivalents.

Considering the mutations with the bioequivalent activity, the NIa protease variants of the present invention may be interpreted as including even the sequences showing substantial identity to those described in the sequence listing.

In a specific embodiment, the TuMV-derived NIa protease variant may be one in which one of the N-glycosylation sites of the NIa protease is substituted with another amino acid. In particular, the N may refer to asparagine (Asn), and the other amino acid may be any one amino acid selected from the group consisting of arginine (Arg), histidine (His), lysine (Lys), aspartic acid (Asp), glutamic acid (Glu), serine (Ser), threonine (Thr), glutamine (Gln), tyrosine (Tyr), alanine (Ala), isoleucine (Ile), leucine (Leu), valine (Val), phenylalanine (Phe), methionine (Met), tryptophan (Trp), glycine (Gly), proline (Pro), and cysteine (Cys).

The N-glycosylation site of the TuMV-derived NIa protease may be N15, N60, or N198 based on the amino acid sequence of SEQ ID NO: 13, but the site is not limited to these mutations as long as it has a mutation that causes the activity of NIa protease.

Additionally, the N-glycosylation site of the TuMV-derived NIa protease may be N70, N115, or N253 based on the amino acid sequence of SEQ ID NO: 31 representing SAS, but the site is not limited to these mutations as long as it has a mutation that causes the activity of NIa protease.

In the present invention, the SAS including the TuMV-derived NIa protease or a variant thereof can inhibit the formation of amyloid β, degrade extracellularly-secreted amyloid β, and also degrade amyloid β that is introduced into the cell from the outside.

In an embodiment of the present invention, in order to determine the specific effect of N-glycosylation occurring in SAS on the activity of SAS, an attempt was made to measure the effect of cleaving the amyloid β precursor protein by preparing a combination of mutations (N70Q, N115Q, or N253Q) in which asparagine (N) at the position for N-glycosylation (N70, N115, or N253) was substituted with glutamine (Q). As a result, the N70Q showed a stronger effect of cleaving the amyloid precursor protein than the conventional SAS. In particular, based on the amino acid sequence of SEQ ID NO: 31 representing SAS, the N70Q type variant was represented by SEQ ID NO: 32, the N115Q type variant by SEQ ID NO: 33, and the N253Q type variant by SEQ ID NO: 34. Additionally, based on the amino acid sequence of SEQ ID NO: 15 representing NIa protease, the N15Q type variant was represented by SEQ ID NO: 19, the N60Q type variant by SEQ ID NO: 20, and the N198Q type variant by SEQ ID NO: 21.

In the present invention, the L2 refers to peptide linker 2. The peptide linker 2 may be a peptide consisting of 1 to 50 amino acids. Preferably, the peptide linker 2 may consist of 1 to 30 amino acids or 2 to 15 amino acids. Specifically, the linker may be a peptide consisting of 1, 2, 3, 4, 5, 6, 7, 8, or 9 amino acids.

For example, the linker may be a polypeptide represented by an amino acid sequence selected from the group consisting of QTDESTLMT (SEQ ID NO: 22), TDESTLMT (SEQ ID NO: 23), QTDESTLM (SEQ ID NO: 24), DESTLMT (SEQ ID NO: 25), QTDESTL (SEQ ID NO: 26), ESTLMT (SEQ ID NO: 27), and QTDEST (SEQ ID NO: 28), but the linker is not limited thereto. The linker merely has a role of linking these monomers, and it has a very limited effect on the structure and impact.

In a specific embodiment, the L2 may be derived from β-secretase. Specifically, the peptide linker 2 may be an amino acid sequence consisting of amino acids at positions 449 to 457 in the amino acid sequence of β-secretase represented by SEQ ID NO: 1 (SEQ ID NO: 22). In the fusion protein of the present invention, the L2 may be present or absent.

The Y may be a transmembrane domain. As used herein, the term "transmembrane domain" refers to a peptide located at a position passing through the cell membrane in the structure of the proteins located on the cell membrane. The transmembrane domain can locate the NIa protease or a fragment thereof or a NIa protease variant inside the endoplasmic reticulum, Golgi apparatus, or endosome, or on the surface of a cell membrane. Additionally, the transmembrane domain has a role of linking NIa protease or a fragment thereof or a NIa protease variant to a domain that transmits an intracellular signal. The transmembrane domain may be derived from any one selected from the group consisting of β-secretase, ELAVL3, NRGN, REEP2, GAD1, PCDHA1, GFAP, S100B, FAM19A1, AQP4, and CLEC2L.

In a specific embodiment, the Y, as a transmembrane domain, may be a polypeptide represented by the amino acid sequence of SEQ ID NO: 29. In the fusion protein of the present invention, the Y may be present or absent.

In the fusion protein of the present invention, the Z may be an intracellular domain. The intracellular domain may be an intracellular signaling domain of a membrane protein.

As used herein, the term "intracellular domain" refers to a region located in the cytoplasm of a membrane protein. In a specific embodiment, the intracellular domain may be a region present in a cell as the C-terminus of the membrane protein.

As used herein, the term "intracellular signaling domain" refers to a region which transmits signals into a cell so as to induce responses (*e.g*., cell activation, release of cytotoxic factors, cytokine production, proliferation, *etc.*) when a signaling receptor on the cell surface recognizes an extracellular signaling substance.

Specifically, the intracellular domain may be derived from β-secretase. The intracellular domain may be an amino acid sequence at positions 479 to 501 in the β-secretase sequence represented by SEQ ID NO: 1, and in a specific embodiment, the intracellular domain may be a polypeptide represented by the amino acid sequence of SEQ ID NO: 30. In the fusion protein of the present invention, the Z may be present or absent.

The fusion protein of the present invention may have a structure of a fusion protein of various combinations depending on the presence or absence of X, L1, L2, Y, or Z of Formula 1 according to the present invention.

In a specific embodiment, the fusion protein of the present invention may be X - L1 - A - L2 - Y - Z; X - L1 - A - Y - Z; X - L1 - A - Y; L1 - A - Y; L1 - A - Y - Z; X - A - Y - Z; X - A - Y; A - Y - Z; X - A; or A - Y, but is not limited thereto.

In a specific embodiment, the fusion protein of the present invention may have a fusion protein structure of Formula 2 below, in which a TuMV-derived β-secretase signal sequence, a TuMV-derived NIa protease, a transmembrane domain, and an intracellular domain are fused.

[Formula 2] X - A - L2 - Y - Z

In the present invention, the fusion protein was expressed as SAS2. SAS2 having the structure of Formula 2 may be a polypeptide represented by the amino acid sequence of SEQ ID NO: 39.

Additionally, the fusion protein of the present invention may have a structure of Formula 3 or Formula 4 below, in which the signal sequence of β-secretase and the pro domain (pro) represented by peptide linker 1 are fused with the NIa variant (D128N) or the NIa variant (N70Q).

[Formula 3] X - L1 - A (D128N) - L2 - Y - Z

[Formula 4] X - L1 - A (N70Q) - L2 - Y - Z

In particular, the NIa variant (D128N) is a negative variant in which the activity is removed by substituting the aspartic acid (D) at position 128 in the catalytic triad with asparagine (N). This variant, based on the amino acid sequence of SEQ ID NO: 15 representing the NIa protease, corresponds to D73N (SEQ ID NO: 18). SAS^{m} having the structure of Formula 3 may be a polypeptide represented by the amino acid sequence of SEQ ID NO: 35. Additionally, the NIa variant (N70Q) was prepared to measure its effect on SAS performance by inactivating one of the N-glycosylation sites of SAS to prevent glycosylation. The SAS^{N70Q} having the structure of Formula 4 above may be C represented by the amino acid sequence of SEQ ID NO: 32.

Additionally, the fusion protein of the present invention may have a structure of Formula 5 below, in which the intracellular signaling domain is removed.

[Formula 5] X - L1 - A - L2 - Y

Additionally, the fusion protein of the present invention may have a structure of Formula 6 below, in which the transmembrane domain Y and the intracellular domain Z are absent.

[Formula 6] X - L1 - A

In particular, the fusion protein (SEC) of Formula 6 above was prepared to be secreted extracellularly by removing the transmembrane domain therein. The SAS^{sec} having the structure of the Formula 6 may be a polypeptide represented by the amino acid sequence of SEQ ID NO: 37.

Additionally, the fusion protein of the present invention may have a structure of Formula 7 below including a GPI targeting signal.

[Formula 7] X - L1 - A - L2 - Y (GPI targeting signal)

Formula 7 above is in a form in which a signal sequence of β-secretase, a prodomain represented by peptide linker 1, NIa protease, and peptide linker 2 are fused with a transmembrane domain Y. In particular, the transmembrane domain may be a GPI targeting signal that is not derived from β-secretase. The SAS^{GPI} having the structure of the Formula 7 may be a polypeptide represented by the amino acid sequence of SEQ ID NO: 38.

In another specific embodiment, the present invention, among the various structures of SAS prepared for the purpose of confirming SAS activity, provides SAS3 which was prepared in such a manner that a murine immunoglobulin Kappa chain V-J2-C signal sequence unrelated to β-secretase was fused to the N-terminus of NIa and a PDGFR transmembrane domain was fused to the C-terminus of NIa. The may be a polypeptide represented by the amino acid sequence of SEQ ID NO: 40.

Additionally, the present invention provides SAS4 prepared by fusing the N-terminus of US9 to the C-terminus of NIa using a type 2 transmembrane domain. The SAS4 may be a polypeptide represented by the amino acid sequence of SEQ ID NO: 41.

### Polynucleotide encoding fusion protein

In another aspect, the present invention provides a polynucleotide encoding a fusion protein according to the present invention.

As used herein, the term "polynucleotide" refers to deoxyribonucleic acid (DNA), ribonucleic acid (RNA), or a DNA/RNA hybrid. The polynucleotide may be single-stranded or double-stranded and may be recombinant, synthetic, or isolated. The polynucleotide includes pre-messenger RNA (pre-mRNA), messenger RNA (mRNA), RNA, genomic RNA (gRNA), plus-stranded RNA (RNA (+)), minus-stranded RNA (RNA (-)), synthetic RNA, synthetic mRNA, genomic DNA (gDNA), PCR amplified DNA, complementary DNA (cDNA), synthetic DNA, or recombinant DNA, but the polynucleotide is not limited thereto.

The polynucleotide may consist of a nucleotide sequence represented by SEQ ID NO: 42. Additionally, the polynucleotide may have about 80%, 90%, 95%, or 99% or more homology to the nucleotide sequence of SEQ ID NO: 42 as long as it can encode the protein of SEQ ID NO: 31. Additionally, the polynucleotide can be codon-optimized.

### Vectors expressing SAS

Additionally, the present invention provides an expression vector which includes the polynucleotide according to the present invention. The polynucleotide is the same as described above.

The polynucleotide can be prepared, engineered, expressed, and delivered using any of a variety of established techniques known and available in the art. For the expression of a target a fusion protein, a polynucleotide encoding the fusion protein may be inserted into an appropriate vector.

As the vector, a variety of vectors known in the art may be used, and depending on the type of the host cell to produce the antigen receptor, expression control sequences (*e.g.,* promoters, terminators, enhancers, *etc.*)*,* sequences for membrane targeting or secretion, *etc.* can be appropriately selected and variously combined according to the purpose. The vector of the present invention includes a plasmid vector, a cosmid vector, a bacteriophage vector, a viral vector, *etc.,* but is not limited to. Suitable vectors include a signal sequence for membrane targeting or secretion or a leader sequence, in addition to expression control elements (*e.g.*, a promoter, an operator, an initiation codon, a stop codon, a polyadenylation signal, an enhancer, *etc.*)*,* and may be prepared in various ways depending on the purpose.

Preferably, the vector may be a viral vector, and the viral vector may be derived from retroviruses, lentiviruses, adenoviruses, adeno-associated viruses (AAV), herpesviruses, poxviruses, baculoviruses, papillomaviruses, vaccinia viruses, and parvoviruses. In an embodiment of the present invention, a lentiviral vector was used.

### Viruses containing nucleic acid encoding SAS

The present invention provides a recombinant virus comprising the polynucleotide according to the present invention. The polynucleotide is the same as described above, and the recombinant virus may be prepared using a plasmid loaded with the polynucleotide according to the present invention.

The recombinant virus may be any one derived from retroviruses, lentiviruses, adenoviruses, adeno-associated viruses (AAV), herpesviruses, poxviruses, baculoviruses, papillomaviruses, vaccinia viruses, and parvoviruses, but is not limited thereto.

In the present invention, the fusion protein that can be expressed by the virus may be a fusion protein in which a signal sequence is removed. In a specific embodiment, the fusion protein may be L1 - A - L2 - Y - Z; L1 - A - Y - Z; L1 - A - Y; L1 - A - Y; A - Y - Z; A - Y; or A, but is not limited thereto.

### Cells expressing SAS

The present invention provides a host cell transfected with an expression vector according to the present invention. In particular, the expression vector is the same as described above. The host cell may be any one selected from the group consisting of *E. coli,* CHO cells, and HEK293 cells, but is not limited thereto. The method of introducing the expression vector of the present invention into a cell can be performed through various methods known in the art.

The methods for introducing the expression vector into cells may be performed using the methods known in the art, for example, transient transfection, microinjection, transduction, cell fusion, calcium phosphate precipitation, liposome-mediated transfection, DEAE Dextran-mediated transfection, polybrene-mediated transfection, electroporation, gene gun, or other known methods for introduction of a nucleic acid into cells (Wu et al., J. Bio. Chem., 267:963-967, 1992; Wu and Wu, J. Bio. Chem., 263: 14621-14624, 1988). However, the methods are not limited thereto.

Transfected host cells may be proliferated *ex vivo* after the introduction of an expression vector. In a specific embodiment, the transfected host cells may be cultured at least for about 1, 2, 3, 4, 5, 6, 7, 8, 9, 10, 11, 12, 13, or 14 days to proliferate, and preferably, may be cultured for 12 to 14 days.

The methods for confirming whether an expression vector has been well introduced into the host cell include, for example, molecular biological assays well known to those skilled in the art, for example, Southern and Northern blotting, RT-PCR and PCR; biochemical assays, for example, detection of the presence or absence of a particular peptide by immunological methods (*e.g.*, ELISAs and Western blotting).

In the present invention, the fusion protein that can be expressed by the host cell may be a fusion protein in which a signal sequence is removed. In a specific embodiment, the fusion protein may be L1 - A - L2 - Y - Z; L1 - A - Y - Z; L1 - A - Y; A - Y - Z; A - Y; or A, but is not limited thereto.

### Pharmaceutical composition containing a vector expressing SAS

In another aspect, the present invention provides a pharmaceutical composition for preventing or treating diseases caused by amyloid β containing the expression vector as an active ingredient.

The pharmaceutically acceptable carriers to be contained in a pharmaceutical composition of the present invention are those commonly used in formulation, which include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, *etc.,* but the pharmaceutically acceptable carriers are not limited thereto. The pharmaceutical composition of the present invention may further contain a lubricant, a wetting agent, a sweetening agent, a flavoring agent, an emulsifying agent, a suspending agent, a preservative, *etc.* in addition to the components described above.

The pharmaceutical composition of the present invention is preferably administered parenterally, for example, may be administered through intravenous administration, subcutaneous administration, or local administration. A suitable dose of the pharmaceutical composition of the present invention varies depending on factors (*e.g.,* a formulation method, an administration method, age, weight, and sex of the patient, degree of disease symptoms, food, administration time, administration route, excretion rate, and response sensitivity) and an ordinarily skilled physician can easily determine and prescribe a dose effective for the intended treatment. In general, the daily dose of the pharmaceutical composition of the present invention may be 0.0001 mg/kg to 100 mg/kg.

The pharmaceutical composition of the present invention may be formulated using a pharmaceutically acceptable carrier and/or excipient according to a method that can easily be performed by those skilled in the art to which the present invention pertains, to be prepared in a unit dose form or prepared by incorporation into a multidose container. In particular, the formulation may be in the form of a solution, suspension, or emulsion in oil or aqueous medium, or may be in the form of an extract, powder, granule, tablet, or capsule, and may additionally include a dispersant or stabilizer.

The disease caused by the amyloid β may be a neurodegenerative disease, and specifically, may be any one selected from the group consisting of Alzheimer's disease, mild cognitive impairment (MCI), mild-to-moderate cognitive impairment, vascular dementia, cerebral amyloid angiopathy, hereditary cerebral hemorrhage, senile dementia, Down syndrome, inclusion body myositis, age-related macular degeneration, and conditions associated with Alzheimer's disease

In the present invention, Alzheimer's disease may include acquired or congenital Alzheimer's disease. Additionally, conditions associated with Alzheimer's disease may include hypothyroidism, cerebrovascular disease, cardiovascular disease, memory loss, anxiety, behavioral dysfunction, neurological or psychological conditions.

The behavioral dysfunction includes apathy, agitation/aggression, depression, irritability/instability, anxiety, or psychosis, but is not limited to. The neurological conditions include Huntington's disease, amyotrophic lateral sclerosis, acquired immunodeficiency syndrome, Parkinson's disease, aphasia, apraxia, agnosia, Pick disease, Lewy body dementia (DLB), altered muscle tone, seizures, sensory loss, visual field deficits, incoordination, gait disturbance, transient ischemic attack or stroke, transient alertness, attention deficit, frequent falls, syncope, susceptibility to tranquilizers, normal pressure hydrocephalus (NPH), subdura hematoma, brain tumor, post-traumatic brain injury, and posthypoxic damage, but the neurological conditions are not limited thereto. The psychological conditions include depression, delusion, illusion, hallucination, sexual dysfunction, weight loss, psychosis, sleep disturbance, insomnia, behavioral disinhibition, degeneration of vision, suicidal ideation, depressive disorder, irritability, anhedonia, seclusion loneliness, and excessive guilt, but the psychological conditions are not limited thereto.

### Pharmaceutical composition containing virus including nucleic acid encoding SAS

Additionally, the present invention provides a pharmaceutical composition for preventing or treating diseases caused by amyloid β containing the recombinant virus according to the present invention as an active ingredient. In particular, the polynucleotide, recombinant virus, diseases caused by amyloid β, ingredients of the pharmaceutical composition, and administration route are the same as described above.

A preferred dose of the pharmaceutical composition containing the recombinant virus of the present invention varies depending on the condition and weight of the individual, the degree of disease, the drug form, and the route and duration of administration, and may be appropriately selected by those skilled in the art. Specifically, the preferred dose may be to administer 1 × 10⁵ to 1 × 10⁸ virus particles, virus units having infectivity (tissue culture infectious dose: TCID50), or plaque forming units (pfu) to a patient, and preferably, to administer 1 × 10⁵, 2 × 10⁵, 5 × 10⁵, 1 × 10⁶, 2 × 10⁶, 5 × 10⁶, 1 × 10⁷, 2 × 10⁷, 5 × 10⁷, 1 × 10⁸, 2 × 10⁸, 5 × 10⁸, 1 × 10⁹, 2 × 10⁹, 5 × 10⁹, 1 × 10¹⁰, 5 × 10¹⁰, 1 × 10¹¹, 5 × 10¹¹, 1 × 10¹², 1 × 10¹³, 1 × 10¹⁴, 1 × 10¹⁵, 1 × 10¹⁶, 1 × 10¹⁷ or higher virus particles, viral units having infectivity, or plaque forming units, and may include various numerical values and ranges therebetween. Additionally, the viral dose is 0.1 mL, 1 mL, 2 mL, 3 mL, 4 mL, 5 mL, 6 mL, 7 mL, 8 mL, 9 mL, 10 mL or greater, including all of the values and ranges therebetween.

Unlike the NIa protease (*i.e.,* a cytoplasmic protein), the fusion protein according to the present invention is a membrane protein, which can exist in a protein secretion pathway including the endoplasmic reticulum and the Golgi apparatus, cell membranes, extracellular-, and protein endocytosis pathway including endosomes and lysosomes. In the present invention, since the fusion protein can cleave an amyloid precursor protein at a site similar to α-secretase, it was named synthetic α-secretase (SAS). SAS can inhibit the formation of amyloid β, degrade extracellularly-secreted amyloid β, and degrade amyloid β that enters cells from the outside. Therefore, the SAS of the present invention can be utilized as a therapeutic agent for various diseases induced by amyloid β.

### Mode for the Invention

Hereinafter, the present invention will be described in detail by the following Examples. However, the following Examples are merely illustrative of the present invention, and the present invention is not limited thereto.

### I. Experimental Preparation and Experimental Methods

### Experimental Method 1. Purification of NIa protease purification and activity measurement using Aβ

For *in vitro* cleavage experiments, 0.5 µM purified NIa was mixed with 2.5 µM monomeric or oligomeric Aβ and buffer (20 mM HEPES (pH 7.4), 10 mM KC1, and 10 mM MgCl₂) and reacted at 25°C for 1 hour or 2 hours. Thereafter, Western blot was performed using an anti-Aβ antibody (4G8) in 15% polyacrylamide gel electrophoresis (PAGE).

### Experimental Method 2. Aβ oligomerization

The synthesized Aβ42 peptide powder was dissolved in 1,1,1,3,3,3-hexafluoroisopropanol (Fluka) to a concentration of 1 mM, evaporated for one day, and stored. Thereafter, the Aβ peptide was dissolved in DMSO to a concentration of 2.5 mM and subjected to sonication for 10 minutes. After dissolving the Aβ in DMEM to a concentration of 100 µM, the oligomers were synthesized by maintaining the resultant at 4°C for 24 hours.

### Experimental Method 3. Preparation of HEK293T cells

Human Embryonic Kidney (HEK) 293T cells were grown in DMEM (Hyclone) medium containing 10% FBS, 100 µg/mL penicillin, and 100 µg/mL streptomycin, and used in the experiment. In most cases, a plasmid vector expressing the APP751 isoform was used as a substrate, and a plasmid expressing a protease was co-transfected using Lipofectamin 2000 (Invitrogen).

### Experimental Method 4. Western blot

When extracting proteins from the cell culture medium, 1 mL of the culture medium was pre-extracted before separation of cell proteins, and then dissolved in the sample buffer by adjusting the concentration. HEK293T cells were washed twice with PBS, dissolved in RIPA buffer containing a protease inhibitor, and proteins were extracted through sonication. The extracted proteins were quantified by the BCA method, and the proteins in the same amount (20 µg) were electrophoresed on 10% or 12% SDS-PAGE for 2 hours. Then, the resultant was transferred to a PVDF membrane. After blocking the PVDF membrane using 5% non-fat milk powder, Western blot was performed by reacting with an appropriate antibody (anti-myc (9B11), anti-Aβ (4G8), anti-sAPPα (2D3), and anti-V5 antibodies were diluted 1:1,000 and used). The primary antibody reaction was performed overnight at 4°C. For the secondary antibody reaction, HRP-conjugated anti-mouse or anti-rabbit antibody was reacted at 1:10,000, and then washed with TBST three times. Thereafter, the presence or absence of the protein was determined by fluorescence through the ECL reaction.

### Experimental Method 5. Treatment of enzyme for removing N-glycosylation

After transfecting HEK293T cells with SAS, whole proteins were extracted. Thereafter, the extracted proteins were denatured by boiling in 2 µL of denaturation buffer (10X) and 20 µL of water at 100°C for 10 minutes. 4 µL of buffer (10X), 4 µL of 10% NP-40, and 2 µL of an enzyme for removing N-glycosylation (PNGaseF or EndoH) were added and reacted in a total volume of 40 µL at 37°C for 1 hour. To confirm the size of SAS, Western blot was performed using an anti-myc antibody (Cell signaling, #2272).

### Experimental Method 6. Amyloid precursor protein (APP)

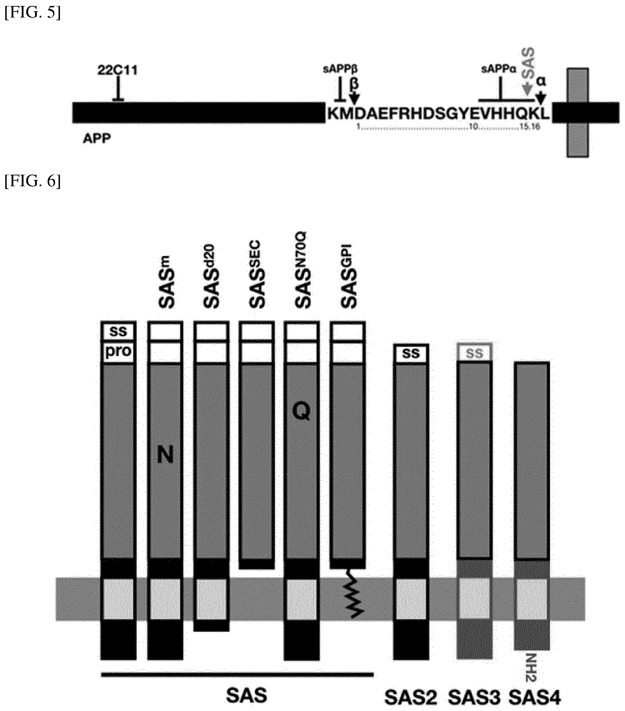

FIG. 5 briefly illustrates APP that generates Aβ, which is one of the causative agents of Alzheimer's disease in humans. Unlike β-secretase that produces amyloid β, α-secretase, which is known to have a neuroprotective action, is located in the middle of Aβ and acts predominantly to form sAPPα, and as a result, the formation of Aβ is reduced. Although SAS is not the exact position of α-secretase, it can produce an effect similar to that of α-secretase by cleaving between glutamine-lysine located one amino acid upstream. Additionally, since SAS does not share substrate specificity with α-secretase, problems caused by overexpression of α-secretase can be prevented. In the present invention, the activity of SAS was measured using the sAPPα antibody, and the antibody, as the antibody acting only on the C-terminus newly created by cleavage, effectively showed α-secretase activity.

### Experimental Method 7. Preparation of 5XFAD mice and gene transfer using adeno-associated virus 9 (AAV9)

5XFAD mice for Alzheimer's model were purchased from Jackson Lab and then used by crossbreeding. Both negative littermate (NL) and transgenic (TG) mice obtained through crossbreeding were used in the experiment. A virus was injected into the mice using stereotaxic injection at the age of 4 months, their behavioral experiments were performed at the age of 6 months, and their brain tissue was extracted by euthanasia at the age of 9 months. As for the virus, AAV9-EGFP expressing green fluorescent protein and AAV9-SAS expressing SAS were used in the experiment. A total of 4 experimental groups were prepared by injecting EGFP and SAS viruses into NL and TG mice. For each experimental group, 7 mice in the NL-EGFP group, 6 mice in the NL-SAS group, 6 mice in the TG-EGFP group, and 8 mice in the TG-SAS group were used. For virus injection, after anesthesia with ketamine and xylazine, the mice were fixed onto a stereotaxic frame, their scalp was incised, and a hole was drilled into the skull at the location where the virus was to be injected. 10 µL of each virus was injected into both lateral ventricles of cerebrum. The drug infusion rate was set to 2 µL/min, and after waiting for 15 minutes upon completion of the injection, the needle was slowly withdrawn.

### Experimental Method 8. Open field test

Two days before this experiment, the mice were moved to the room where the experiment was conducted and stabilized. The day before the experiment, the mice were acclimatized by placing them in the center of an opaque test box of 36 cm ^{∗} 36 cm ^{∗} 40 cm (width ^{∗} length ^{∗} height) and allowing them to move freely for 5 minutes. On the day of the experiment, the mice were placed in the center of the test box and the total movement distance was measured by recording their movement for 20 minutes.

### Experimental Method 9. Novel object recognition test

On the first day, two identical objects were placed at regular intervals in the center of an opaque test box of 36 cm ^{∗} 36 cm ^{∗} 60 cm (width ^{∗} length ^{∗} height), and a mouse was placed in the center of the objects and allowed to move freely for 8 minutes. The next day, one of the two objects was changed to another object, and the mouse was placed in the middle of the objects, and the time taken for the mouse to search for each object was measured for 8 minutes. Preference index (%) was measured through the equation (time taken to search for a new object)/(time taken to search for all objects) ^{∗} 100.

### Experimental Method 10. Morris water maze test

Two markers were attached to the wall of a circular pool of water with a diameter of 120 cm at regular intervals, and a platform was placed 1.5 cm below the water surface near one of them so that mice could climb. The time taken for the mouse to climb the platform within 60 seconds was analyzed by placing the mouse in a different quadrant except for the place where the platform was located 4 times a day for 4 days. When the mouse arrived at the platform or the mouse was transferred to the platform, if it did not climb onto the platform within the time limit, the mouse was left on the platform for 10 seconds to memorize the location. On the fifth day, the platform was removed and the mouse was allowed to swim freely for 120 seconds, and the time and number of times it reached each quadrant and the platform were measured. On the 7th and 8th days, after changing the position of the platform in the opposite direction, the mouse was released from the position where the platform was located at the beginning, and the time for the mouse to find the position of the changed platform within 60 seconds 4 times a day was measured.

### Experimental Method 11. Immunohistochemical analysis for observation of amyloid plaques

After euthanizing the mouse, it was subjected to perfusion using PBS, and the brain was extracted. Thereafter, the tissue was fixed with 4% PFA for one day, washed with PBS for one day, and stored in 30% sucrose for 3 to 5 days. The entire process was performed at 4°C. Then, the tissue was embedded with an OCT compound, and frozen sections were prepared using a cryotome. Thereafter, the tissue was washed with PBS, fixed in 4% PFA for 20 minutes, subjected to permeabilization using 0.1% Triton X-100 solution, and blocked using 5% goat serum and a 5% BSA solution. Then, antigen retrieval was performed using a 0.1 M citrate [pH 6.0] solution, and the Aβ antibody (MOAB-2) was diluted 1:1,000 and reacted overnight. After washing with PBS, the Alexa 594-conjugated anti-mouse antibody was diluted 1:1,000 and Hoechst 33342 dye was diluted 1:2,000, and they were reacted for 4 hours. The resultant was washed with PBS and mounted. Then, the images of the hippocampus of the brain were obtained using a fluorescence microscope, and the hippocampus was subjected to quantitative analysis was performed sing ImageJ software (https://imagej.nih.gov/ij/).

### Example 1. Preparation of SAS

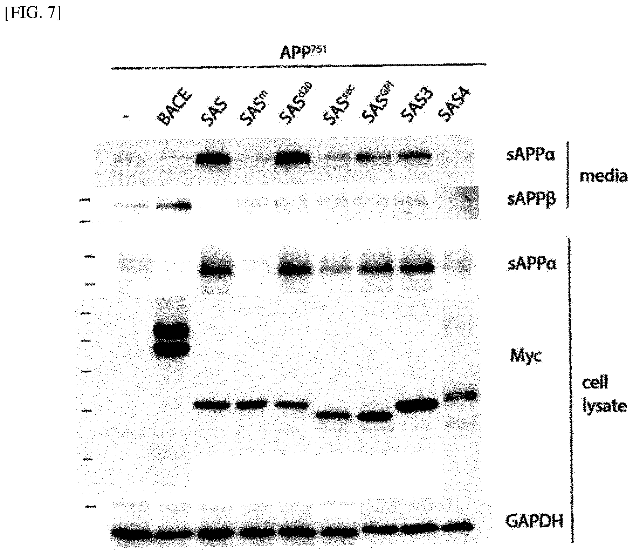

SAS was prepared by fusing the N-terminus and C-terminus of NIa with the pro domain and the transmembrane domain of β-secretase. In order to find effective SAS activity, various versions of SAS were prepared through gene synthesis (https://www.idtdna.com), and the experiment was performed by inserting the prepared SAS into the pcDNA4 vector suitable for intracellular expression using *Eco*RI and *Xho*I*.* SAS (SAS^{d20}, SAS^{SEC}, SAS^{GPI}, *etc.*) was prepared by fusion of signal sequences and prodomains with NIa, and SAS2 was prepared using only a β-secretase signal sequence (signal sequence, ss). SAS3 was prepared together with NIa by linking the V-J2-C signal sequence of the murine immunoglobulin Kappa chain, which is independent of β-secretase, to the N-terminus while linking the PDGFR transmembrane domain to the C-terminus. SAS4, which is a type2 transmembrane domain, was prepared by fusing the N-terminus of US9 with NIa. The SAS structures are shown in FIG. 6, and their activity is shown in FIG. 7. As shown in FIG. 7, among the various versions of SAS, the SAS^{d20} activity was the most outstanding.

In order to study the properties of SAS, various variant forms were prepared. SAS^{m} is a negative variant (negative mutant) of the catalytic triad of NIa in which aspartic acid (D) is replaced with asparagine (N) to abolish activity. SAS^{d20}, which is also a SAS fragment, was prepared to confirm the effect of the localization signal of β-secretase on SAS activity by removing 20 amino acids corresponding to the C-terminus of β-secretase. In particular, the SAS^{d20} may be a polypeptide represented by the amino acid sequence of SEQ ID NO: 36. Meanwhile, in the intracellular domain corresponding to Z of the fusion protein according to the present invention, 1, 2, 3, 4, 5, 6, 7, 8, 9, 10, 11, 12, 13, 14, 15, 16, 17, 18, 19, or 20 amino acid residues may be deleted consecutively. In the case of SAS^{SEC}, it was prepared to be secreted extracellularly by removing the transmembrane domain, and through SAS^{GPI}, the activity of SAS was tested after binding to the cell membrane by attaching a GPI targeting signal thereto although the transmembrane domain was removed. Finally, by way of using SAS^{N70Q}, one of the N-glycosylation sites of SAS was inactivated to prevent glycosylation, and the effect on SAS performance was measured.

### II. Confirmation of SAS activity

### Experimental Example 1. Confirmation of amyloid β-degrading activity of NIa and fragments thereof

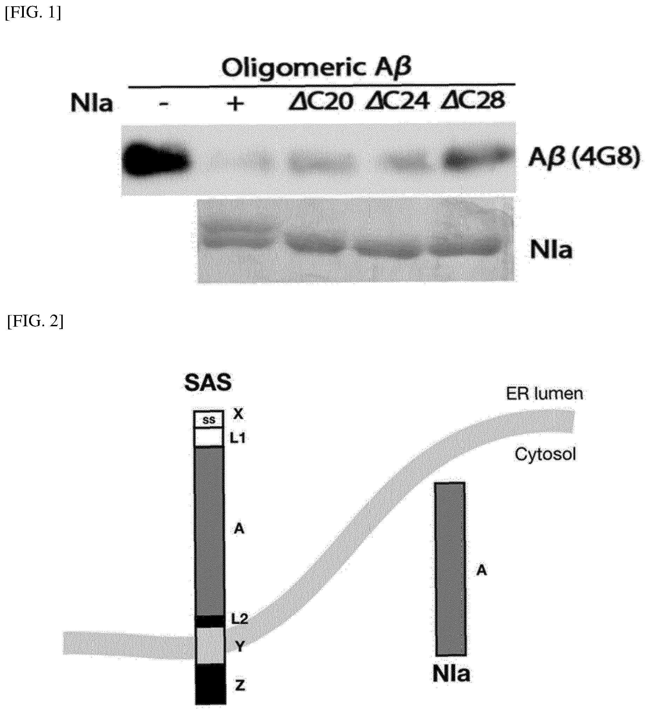

After expressing and purifying TuMV-derived NIa protease and various fragments in *E. coli,* their protease activity was confirmed using amyloid β as a substrate. The results of examining the amount of amyloid β under each condition are shown in FIG. 1. As shown in FIG. 1, it was confirmed that amyloid β disappeared after treatment with NIa and its fragment for 1 hour. Additionally, as self-cleavage at the C-terminus was reported in several studies on TuMV NIa, self-cleavage activity was confirmed by the appearance of two bands after protein purification in this experiment.

Experiments were performed to determine whether the protease in which the self-cleavage occurred also had its activity and whether the activity to degrade amyloid β was maintained even when more amino acids were removed. As a result, it was confirmed that NIaΔ20 (in which 20 amino acids were removed from the C-terminus) and NIaΔ24 (in which 24 amino acids were removed) showed activity similar to normal NIa, and NIaΔ28 (in which 28 amino acids were removed) showed significantly reduced activity (FIG. 1).

### Experimental Example 2. Measurements of SAS activity

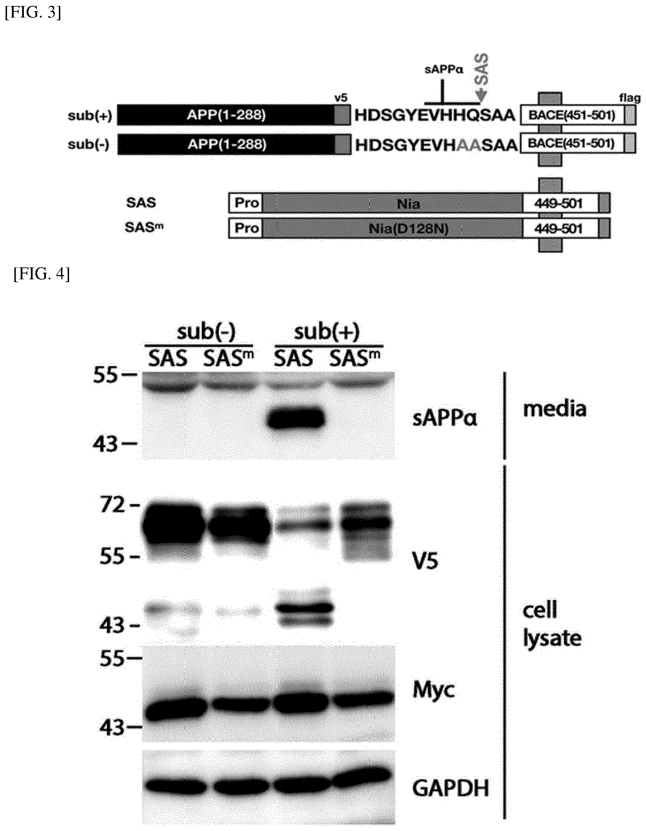

To measure the SAS activity in cells, substrate sub (+) and substrate sub (-) designed to prevent SAS from acting on the substrate were prepared. In order to block the results from being disturbed by α-secretase and γ-secretase that may exist in the cell, the substrate sequence that can act on α-secretase and γ-secretase was removed from sub (+), and the sequence optimized for SAS VXHQ/S was used. In sub (-), both the PI (histidine) and P2 (glutamine) moieties were substituted with alanine so that SAS cannot function. For the N-terminal part, an APP N-terminus with an appropriate size was used so that it could be secreted out of the cell. Additionally, for easy detection of SAS activity, the V5 epitope was placed at the N-terminus and the Flag epitope was placed at the C-terminus. As the C-terminal portion, the C-terminus of β-secretase was used as in SAS so that the protein containing the substrate could be located at the same location in the cell as the SAS (FIG. 3). The activity of SAS confirmed by the substrate is shown in FIG. 4.

### Experimental Example 3. Confirmation of SAS activity in HEK293 cells

Human kidney-derived cells (HEK293T cells) were co-transfected with APP (a substrate) and vectors expressing respective proteases, and then SAS activity was measured by Western blot using the sAPPα antibody. The results are shown in FIG. 7.

As shown in FIG. 7, when only APP was transfected, the sAPPα band did not appear in the media or in the cell lysate, thus confirming that there was no or weak intrinsic α-secretase activity. Additionally, when β-secretase alone was acted on APP, it did not show an effect of increasing sAPPα, but the SAS prepared by fusion showed a distinct sAPPα band in the media and in the cell lysate. In particular, the activity of β-secretase was confirmed by the sAPPβ band.

However, in the case of the negative variant, SAS^{m}, the sAPPα band did not appear, thus confirming that the sAPPα band appeared due to the catalytic activity of SAS. When the 20 amino acids at the C-terminus, which are thought to be important for the intracellular localization of SAS, were removed (SAS^{d20}), the activity of SAS was not significantly affected. From this result, it was found that SAS activity is occurring in the process of being secreted into the cell membrane through the endoplasmic reticulum and Golgi apparatus. Considering that when SAS was prepared to be secreted immediately (SAS^{sec}) by removing the transmembrane domain, its activity was significantly reduced, whereas when it was placed again on the cell membrane through GPI (SAS^{GPI}), its activity was restored, it was found that SAS activity mainly occurs near the cell membrane.

Although the activity was the best when the protein sequence of β-secretase was used, significant activity was also shown when a sequence derived from another protein was used (SAS3), thus confirming that the N-terminus and C-terminus of β-secretase do not significantly affect the activity of SAS. In the case of SAS4, which was designed to have the N-terminus positioned inside the cell, did not increase sAPPα. Therefore, it was found that the topology of SAS is important (FIG. 7).

### Experimental Example 4. Confirmation of N-glycosylation of SAS and effect of glycosylation on activity

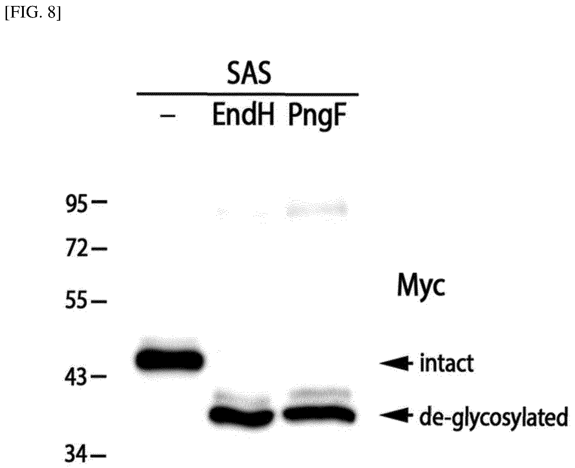

EndoH and PNGaseF (PngF), which are enzymes capable of specifically removing N-glycosylation, were reacted with SAS expressed in HEK293T cells, followed by Western blot. The results are shown in FIG. 8. As shown in FIG. 8, both EndoH and PNGaseF acted on SAS to significantly reduce their size, and considering that the reduced size was almost identical to 37 kD estimated by the amino acid sequence, it was confirmed that SAS was being N-glycosylated in the cell.

As a result of confirming the protein sequence of SAS, N70, N115, and N253 were identified as positions capable of N-glycosylation. It has been reported that when TEV NIa, which is an intracellular protease similar to TuMV, was secreted out of the cell, glycosylation, which did not occur in the original cell, proceeded, and as a result, it affected the activity of TEV Nia (Cesaratto *et al.,* 2015). Therefore, in order to examine how glycosylation in SAS affects the activity of SAS, a combination of mutants in which asparagine at the N-glycosylation position was substituted with glutamine were prepared, allowed to express together with APP, and their APP-cleaving activity was measured.

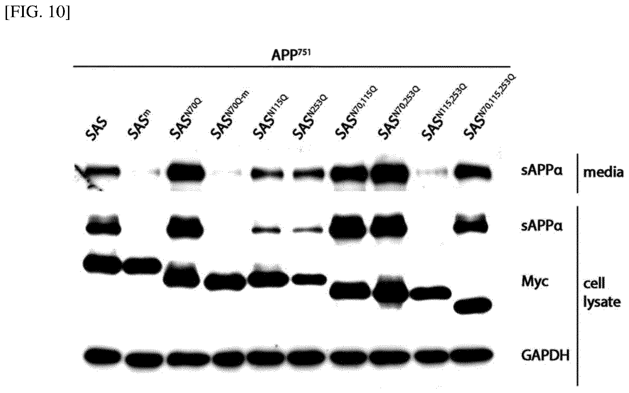

Surprisingly, the first glycosylation site mutation, SAS^{N70Q}, showed stronger activity than the original SAS. Such an effect was also seen in SAS^{N70Q,N115Q}, SAS^{N70Q,N253Q}, *etc.* which include N70Q, but the activity was halved or disappeared in the SAS^{N70Q,N115Q,N253Q} mutants in which glycosylation was completely eliminated. From these results, it could be speculated that the first glycosylation (N70) had an effect to interfere with the activity of SAS, and the other two glycosylation (N115, N253) were neutral or helpful for the activity (FIG. 10).

### Experimental Example 5. SAS preparation and activity measurement using NIa protease derived from potyvirus other than TuMV

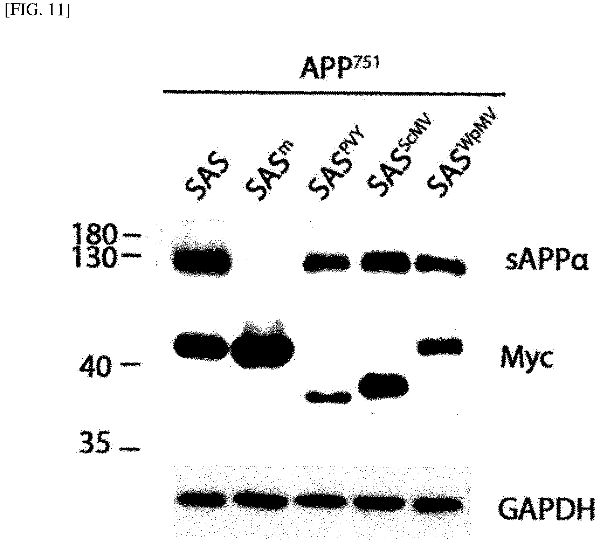

It was expected that the NIa protease of other viruses belonging to the genus potyvirus could be used for SAS production, in addition to the TuMV-derived NIa protease. In particular, it was expected that SAS could be prepared using other potyvirus-derived Nia proteases capable of specifically cleaving the valine-X-histidine-glutamine (V-X-H-Q) region, in a similar manner to TuMV-derived NIa protease. As a result of analyzing the potyvirus genome, about one hundred potyvirus NIa proteases were included in this category. In order to test this possibility, part of the N-terminus and the C-terminus were removed from each NIa protease, which was derived from *Potato Virus Y* (PVY, SEQ ID NO: 43), *Sunflower cholorotic Mottle virus* (ScMV, SEQ ID NO: 45), and *Wild Potato mosaic virus* (WpMV, SEQ ID NO: 47) (*i.e.,* PVY NIa NΔ8CΔ26, SEQ ID NO: 44; ScMV NIa NΔ8CΔ26, SEQ ID NO: 46; and WpMV NIa NΔ8CΔ28, SEQ ID NO: 48) to suit SAS, and SAS was prepared as shown in FIG. 2 using the same. These new SASs were named SAS^{PVY} (SEQ ID NO: 49), SAS^{ScMV} (SEQ ID NO: 50), and SAS^{WpMV} (SEQ ID NO: 51), respectively. After co-transfection of these SASs and APP into HEK293T cells, the activity of these SASs was measured by Western blot using the sAPPα antibody. As shown in FIG. 11, these SASs showed APP cleavage activity similar to that of SAS which was prepared using TuMV-derived NIa protease.

### Experimental Example 6. Confirmation of memory recovery effect of SAS in 5XFAD mice

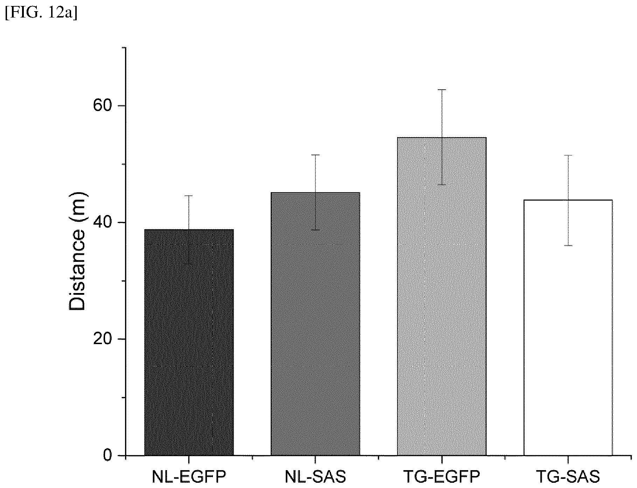

After expressing EGFP and SAS in NL and TG mice using AAV9 virus, respectively, novel object recognition (NOR) and Morris water maze (MWM) behavioral experiments were performed to observe memory ability. The results are shown in FIGS. 12a to 12e.

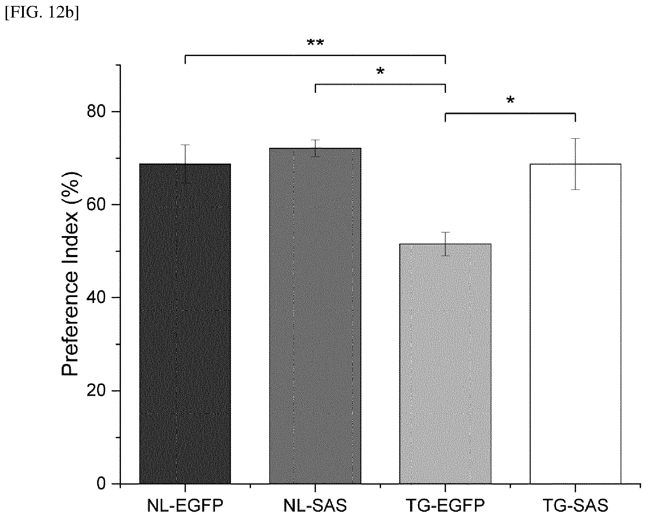

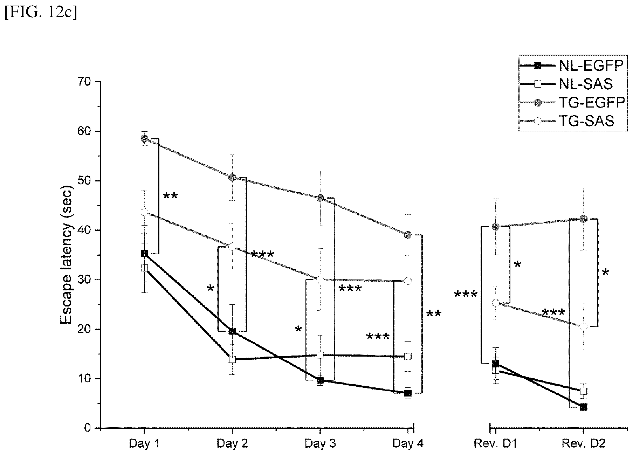

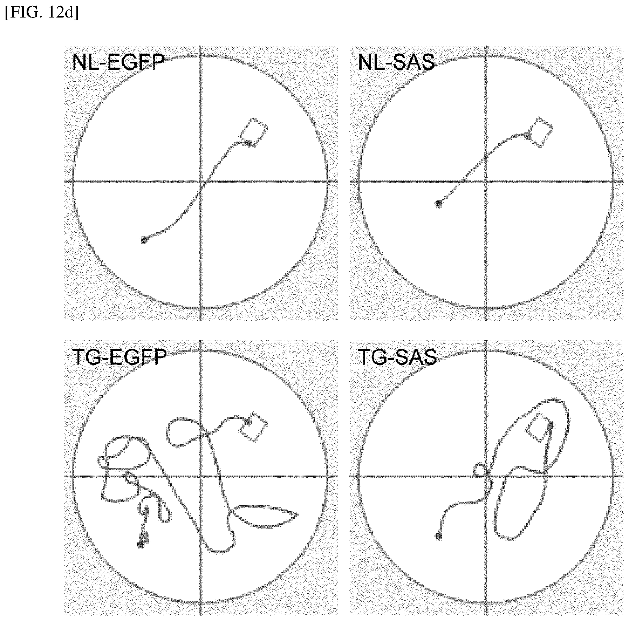

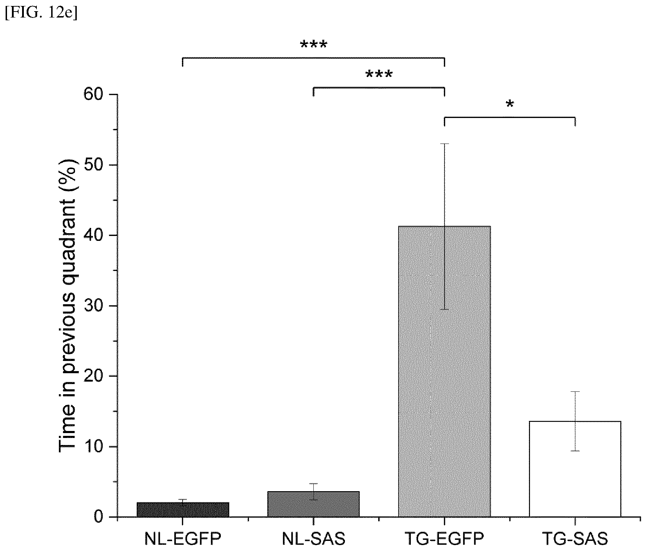

Referring to FIG. 12a, as a result of confirming that basic motility was normal by measuring the distance the mouse freely moved for a certain period of time with the open field test (OFT), SAS showed no effect on the basal exercise ability of the mice. Additionally, through FIG. 12b, the memory ability for a new object was observed by measuring the ratio of the search time for the existing object and the new object, and thereby confirmed that the memory ability for a new object, which had been decreased in the TG-EGFP group, was restored by expressing SAS. Additionally, reviewing FIG. 12c, it was confirmed that although the TG-EGFP group showed a difficulty in learning the platform position, this learning ability was restored through SAS expression. Further, through the reversal test of FIG. 12c (a test with the platform position reversed), FIGS. 12d, and 12e, it was confirmed that cognitive flexibility was significantly restored in the TG-SAS group when the platform position was changed compared to the TG-EGFP group. That is, through this experiment, it was confirmed that the expression of SAS using AAV9 had a therapeutic effect to restore the memory loss of 5XFAD mice.

### Experimental Example 7. Confirmation of plaque removal activity of SAS in 5XFAD mice

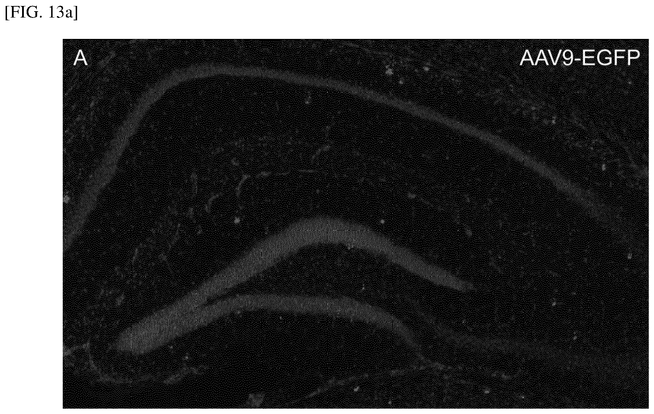

After extracting the brains of the mice used in Experimental Example 6, amyloid plaques were observed through immunohistochemical analysis, and the results are shown in FIGS. 13a to 13d.

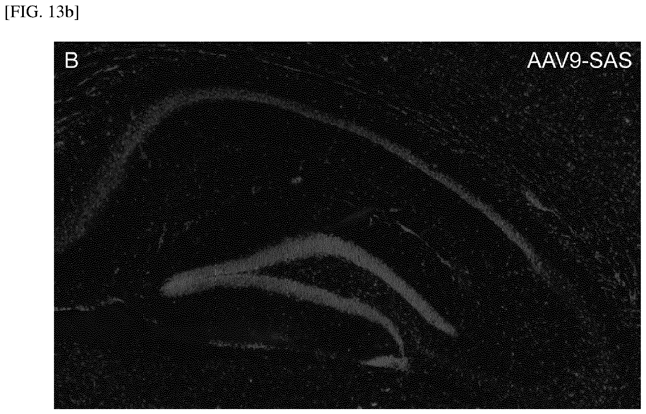

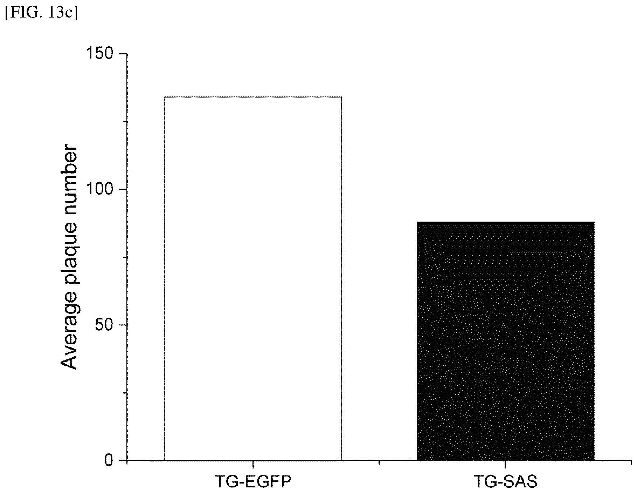

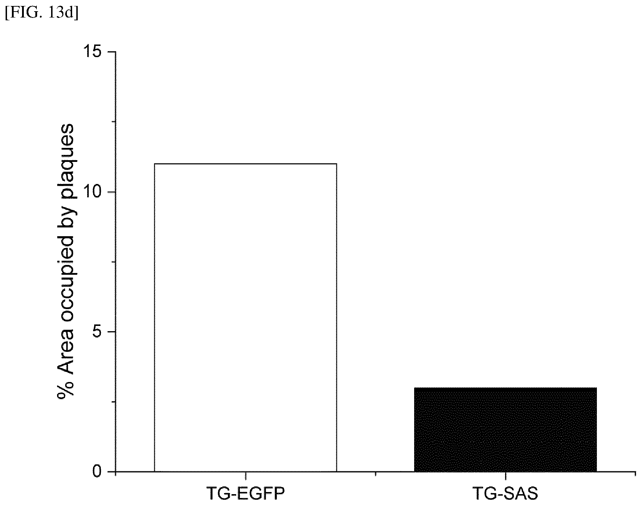

As shown in FIGS. 13a and 13b, it was confirmed that plaques stained red with Aβ antibody were accumulated in the hippocampus, which controls memory ability, in the brain of the mice of the TG-EGFP group, and these plaques were reduced in the TG-SAS group. Additionally, through the quantitative analysis of FIGS. 13c and 13d, it was confirmed that the TG-SAS group showed a decrease in both the number and area of plaques compared to the TG-EGFP group. Therefore, it was confirmed that the expression of SAS through AAV9 showed a therapeutic effect on reducing plaques accumulated in the brain of 5XFAD mice.

## Claims

1. A fusion protein of Formula 1 below:
[Formula 1] (X)ₘ- (L1)ₙ - A - (L2)ₒ - (Y)p - (Z)q
wherein in Formula 1 above,
X is a signal sequence;
L1 is peptide linker 1;
A is a nuclear inclusion a (NIa) protease or a fragment thereof, or a variant thereof;
L2 is peptide linker 2;
Y is a transmembrane domain;
Z is an intracellular domain; and
m, n, o, p, and q are each 0 or 1.

2. The fusion protein of claim 1,
wherein the signal sequence is derived from β-secretase.

3. The fusion protein of claim 1,
wherein the signal sequence is a polypeptide having the amino acid sequence of SEQ ID NO: 3.

4. The fusion protein of claim 1,
wherein the peptide linker 1 is a peptide consisting of 3 to 24 amino acids.

5. The fusion protein of claim 1,
wherein the peptide linker 1 is derived from β-secretase.

6. The fusion protein of claim 1,
wherein the peptide linker 1 is a polypeptide represented by amino acids of SEQ ID NOS: 5 to 11.

7. The fusion protein of claim 1,
wherein the NIa protease is derived from the family *Potyviridae.*

8. The fusion protein of claim 1,
wherein the NIa protease has an activity to degrade amyloid β.

9. The fusion protein of claim 1,
wherein the NIa protease is a polypeptide represented by the amino acid sequence of SEQ ID NO: 12.

10. The fusion protein of claim 1,
wherein the NIa protease variant is one in which one of the N-glycosylation sites of the NIa protease is substituted with another amino acid.

11. The fusion protein of claim 10,
wherein the another amino acid is any one selected from the group consisting of arginine (Arg), histidine (His), lysine (Lys), aspartic acid (Asp), glutamic acid (Glu), serine (Ser), threonine (Thr), glutamine (Gin), tyrosine (Tyr), alanine (Ala) , isoleucine (Ile), leucine (Leu), valine (Val), phenylalanine (Phe), methionine (Met), tryptophan (Trp), glycine (Gly), proline (Pro), and cysteine (Cys).

12. The fusion protein of claim 10,
wherein the N-glycosylation site is N70, N115, or N253 in the amino acid sequence of SEQ ID NO: 31.

13. The fusion protein of claim 1,
wherein the transmembrane domain is derived from any one selected from the group consisting of BACE, ELAVL3, NRGN, REEP2, GAD1, PCDHA1, GFAP, S100B, FAM19A1, AQP4, and CLEC2L.

14. The fusion protein of claim 1,
wherein the transmembrane domain is a polypeptide represented by the amino acid sequence of SEQ ID NO: 29.

15. The fusion protein of claim 1,
wherein the intracellular domain is derived from β-secretase.

16. The fusion protein of claim 1,
wherein the intracellular domain is a polypeptide having the amino acid sequence of SEQ ID NO: 30.

17. A polynucleotide encoding the fusion protein according to claim 1.

18. The polynucleotide of claim 17,
wherein the polynucleotide is a polynucleotide having the nucleotide sequence of SEQ ID NO: 42.

19. An expression vector comprising the polynucleotide of claim 17.

20. A recombinant virus comprising the polynucleotide of claim 17.

21. A host cell transfected with the expression vector of claim 19.

22. The host cell of claim 21,
wherein the host cell is any one selected from the group consisting of *E. coli,* CHO cells, and HEK293 cells.

23. A pharmaceutical composition for preventing or treating diseases caused by amyloid β comprising the expression vector according to claim 19 as an active ingredient.

24. The pharmaceutical composition of claim 23,
wherein the disease caused by amyloid β is selected from the group consisting of Alzheimer's disease, mild cognitive impairment (MCI), mild-to-moderate cognitive impairment, vascular dementia, cerebral amyloid angiopathy, hereditary cerebral hemorrhage, senile dementia, Down syndrome, inclusion body myositis, age-related macular degeneration, and conditions associated with Alzheimer's disease.

25. A pharmaceutical composition for preventing or treating diseases caused by amyloid β comprising the recombinant virus of claim 20 as an active ingredient.

26. The pharmaceutical composition of claim 25,
wherein the virus is any one selected from the group consisting of retroviruses, lentiviruses, adenoviruses, adeno-associated viruses (AAV), herpesviruses, poxviruses, baculoviruses, papillomaviruses, vaccinia viruses, and parvoviruses.

27. A method for preventing or treating diseases caused by amyloid β, comprising administering the composition according to any one of claims 23 to 26 to an individual.
